# EUROPEAN PATENT SPECIFICATION

(11) **EP 2 330 114 B1**
(45) Date of publication and mention of the grant of the patent: **16.08.2017**
(21) Application number: 09808062.5
(22) Date of filing: 18.08.2009
(51) Int. Cl.: C07K 14/00, C07K 1/113, C07K 1/16, C07K 1/107

(54) **GLYCOPROTEIN PRODUCTION METHOD AND SCREENING METHOD**
VERFAHREN ZUR GLYCOPROTEINHERSTELLUNG UND SCREENINGVERFAHREN
PROCÉDÉ DE PRODUCTION DE GLYCOPROTÉINE ET PROCÉDÉ DE CRIBLAGE

(30) Priority: 19.08.2008 JP 2008211144; 11.02.2009 JP 2009029206
(43) Date of publication of application: 08.06.2011
(73) Proprietor: Glytech, Inc., Kyoto 600-8813 (JP)
(72) Inventor: KAJIHARA, Yasuhiro, Toyonaka-shi Osaka 560-0054 (JP); FUKAE, Kazuhiro, Tokusima-shi Tokushima 771-0193 (JP)
(74) Representative: Niizuma, Yo
(86) International application number: PCT/JP2009/003932
(87) International publication number: WO 2010/021126

(56) References cited:
- WO-A1-94/15968
- WO-A2-2005/055946
- JP-A- 2008 050 344
- JP-T- 2008 520 190
- NAOKI YAMAMOTO ET AL: "Chemical Synthesis of a Glycoprotein Having an Intact Human Complex-Type Sialyloligosaccharide under the Boc and Fmoc Synthetic Strategies", JOURNAL OF THE AMERICAN CHEMICAL SOCIETY, vol. 130, no. 2, 1 January 2008 (2008-01-01), pages 501-510, XP055008227, ISSN: 0002-7863, DOI: 10.1021/ja072543f
- YASUHIRO KAJIHARA ET AL.: 'Hito Fukugogata Tosa o Motsu Kogata To Tanpakushitsu no Kagaku Gosei' DAI 49 KAI SYMPOSIUM ON THE CHEMISTRY OF NATURAL PRODUCTS, SYMPOSIUM PAPERS 2007, pages 637 - 642, XP008143260
- YASUHIRO KAJIHARA ET AL.: 'Yuki Gosei ni yoru Tosa Oyobi To Tanpakushitsu no Gosei Gijutsu no Doko' BIOTECHNOLOGY JOURNAL vol. 6, 2006, pages 299 - 305, XP008143265
- SHUN SASAOKA ET AL.: 'Tosaka Ovomucoid no Gosei Kenkyu' 87TH ANNUAL MEETING OF CHEMICAL SOCIETY OF JAPAN IN SPRING KOEN YOKOSHU 2 2007, pages 1231 - 1H4-47, XP008143259

## Description

### [Technical Field]

The present invention, as defined in the claims, relates to a method for producing a glycoprotein having uniform amino acid sequence, sugar chain structure, and higher order structure.

### [Background Art]

Recently, a research on the use of a glycoprotein as various medicines has been carried out. The sugar chain moiety of a glycoprotein serves a function of imparting resistance to the glycoprotein against a protease so as to delay the glycoprotein being metabolized out of the blood, a function of being a signal governing transportation of the glycoprotein to organelles within a cell, and the like. Accordingly, addition of an appropriate sugar chain enables control of the blood half-life and the intracellular transportation of a glycoprotein.

Erythropoietin (EPO) is a representative example showing that a sugar chain affects the physiological activity of a glycoprotein. This glycoprotein is a hematocyte differentiation hormone, which serves a function of maintaining the erythrocyte count in the peripheral blood by acting on erythroid progenitor cells to promote their proliferation and differentiation. A study on the correlation between the sugar chain structure of EPO and its physiological activity revealed that although EPO lacking a sugar chain still exhibited a physiological activity in vitro, it was readily excreted through the kidney in vivo, failing to exhibit a sufficient physiological activity.

Further, when a glycoprotein has an imperfect sugar chain, and also when a different sugar chain is bound to a glycoprotein, such a glycoprotein may be eliminated from the blood upon recognition by macrophages and the like present in the blood.

Accordingly, when a glycoprotein is used as a pharmaceutical product, it is desirable that each protein have a uniformly-structured sugar chain bound to the same position.

Conventionally, as a production method of a glycoprotein, a method of enzymatically adding a sugar to a protein is widely used. However, in this method, a uniform sugar chain cannot be added, and also, it is difficult to uniformly apply modification and trimming after addition of a sugar chain.

Also, while a protein preparation is generally evaluated based on its titer, there is a possibility that preparations with the same titer may contain proteins with various sugar chain structures, which may cause variation in the blood half-life or cause a problem in terms of quality control.

The present inventors have so far developed a method enabling production of a relatively large amount of a glycoprotein having uniform amino acid sequence and sugar chain from an amino acid having an amino group protected with a fat-soluble protecting group and an asparagine-linked sugar chain (for example, refer to Patent Literature 1). Further, they have developed an aminated complex-type sugar chain derivative and a glycoprotein capable of maintaining sufficient blood concentrations (for example, refer to Patent Literature 2). Either of the above glycoproteins is anticipated to be utilized as a pharmaceutical product.

Meanwhile, to be used as a pharmaceutical product, a glycoprotein having a constant physiological activity needs to be produced. Not only the amino acid sequence and the sugar chain structure but also the higher order structure of the protein moiety are considered to be closely related the function of a glycoprotein.

The higher order structure of a protein is stabilized by a hydrogen bond, an ionic bond, and a hydrophobic interaction between amino acid residues as well as an S-S bond between cysteine residues, and the like, and most proteins each have a unique higher order structure. However, bonds other than an S-S bond are relatively weak, and thus a higher order structure of the protein is destroyed by relatively mild heating, pressure, and the like, by which the physiological activity of the protein is reduced and lost. This is called protein denaturation. Also, particularly when the amino acid chain is long, because more than one structures providing the minimum point of energy are generated, an abnormal higher order structure (misfolding) may occur. In that case also, the protein activity is reported to be changed or lost.

Based on the foregoing facts, it is generally considered that a correct higher order structure is essential in order for a protein to exhibit its function, and when proteins are folded, they are separated into either a correctly-folded protein having a physiological activity or a misfolded protein lacking a physiological activity.

Although various researches have been conducted on the relationship between the higher order structure of a protein and the physiological activity, no report has been made as to how a sugar chain can impact the folding and the physiological activity of an artificially-synthesized glycoprotein.

The present inventors synthesized glycoprotein fragments according to the method of Patent Literature 1 and linked them to other peptide fragments by Native Chemical Ligation (NCL) to synthesize monocyte chemotactic protein-3. The monocyte chemotactic protein-3 thus synthesized was folded and the position of a disulfide bond was confirmed by chymotrypsin treatment. As a result, it was revealed that while the disulfide bond was formed at a correct position in approximately 90% of glycoprotein, the disulfide bond was formed at a position different from the normal position in approximately 10% of glycoprotein (Non Patent Literature 1).

However, according to the above literature, such a variation in the position of disulfide bond is not observed when two or more different kinds of glycoproteins are folded. Considering a possibility of disulfide bond reformation during the chymotrypsin treatment, a possibility that two or more kinds of folding were not occurring but the chymotrypsin treatment merely produced two or more kinds of results cannot be ruled out. Accordingly, needless to say, no study has been carried out either on a difference in the physiological activity of a glycoprotein having a disulfide bond formed at a correct position and that of a glycoprotein having a disulfide bond formed at a position different from the normal position.

Further, ovomucoid protein is one of the glycoproteins whose function and structure have been relatively well studied. Ovomucoid protein is a kind of protein contained in egg white with a molecular weight of approximately 28, 000. It has three domains within the molecule, each of which has an inhibitory activity on different proteases. Particularly, the third domain is studied in detail since it exhibits an inhibitory activity even by itself. So far, the structure of the third domain derived from 100 or more kinds of birds has been reported, and its conformation has been elucidated also by X-ray crystallography.

As to the stereostructure of a chemically-synthesized ovomucoid third domain, for example, it is reported that an ovomucoid third domain with modified peptide scaffold is synthesized by NCL and then analyzed by x-ray crystallography (refer to Non Patent Literature 2).

Further, it is also reported that, when an ovomucoid third domain synthesized by stepwise synthesis and NCL was folded, a result strongly suggestive of the third domain being correctly folded was obtained through heat stability analysis (refer to Non Patent Literature 3).

### [Patent Literature]

[Patent Literature 1] WO2004/005330
[Patent Literature 2] WO2005/010053

### [Non Patent Literature]

[Non Patent Literature 1] Yamamoto et al. , Journal of American Chemical Society, 2008, 130, 501-510
[Non Patent Literature 2] Bateman et al. , Journal of Molecular Biology (2001) 305, 839-849
[Non Patent Literature 3] Lu et al., Journal of American Chemical Society, 1996, 118, 8518-8523

### [Summary of Invention]

### [Problem to be solved by the invention]

However, according to the aforementioned conventional technology, no sugar chain is added to a synthesized protein, and thus how a sugar chain affects the folding and the physiological activity of a glycoprotein has not been known.

In view of the above, one object of the present invention is to provide a glycoprotein having not only a uniform sugar chain-based function such as the blood half-life but also a uniform physiological activity, that is, a glycoprotein having uniform amino acid sequence, sugar chain structure, and higher order structure.

Further, another object of the present invention is to provide a screening method for selecting a glycoprotein having a predetermined activity from among plural kinds of glycoproteins with various intensity of the physiological activity, and to provide a glycoprotein mixture having a desired activity.

### [Means for solving problem]

The present inventors have found that, by synthesizing a third domain of ovomucoid protein having uniform amino acid sequence and sugar chain structure and folding the product thus obtained, a mixture containing plural kinds of higher order structures at a constant ratio can be obtained with good reproducibility. Further, as they separated the resulting product and measured its physiological activity, unlike the conventional understanding, it was confirmed that there were plural kinds of higher order structures that had the same kind of physiological activity at a level considered to be relatively highly active, and although relatively highly active, the activity varied depending on the higher order structure, and that glycoproteins with various higher order structures could be each separated and purified by column chromatography.

Also, they have found that a glycoprotein other than the glycoprotein having a predetermined activity can be converted into the higher order structure that is obtained at a constant ratio as described above by once unfolding it and then refolding it, and thus, a glycoprotein having a higher order structure exhibiting a predetermined activity can be maximally collected by repeating the unfolding/refolding step.

That is, the present invention, as defined in the claims, provides a method for producing a glycoprotein having uniform amino acid sequence, sugar chain structure, and higher order structure, comprising the following steps (a) to (c):
(a) folding a glycoprotein having uniform amino acid sequence and sugar chain;
(b) fractionating the folded glycoprotein by column chromatography; and
(c) collecting a fraction having a predetermined activity.

Preferably, the aforementioned method further include, after the step (c), the steps of:
(d) unfolding a glycoprotein contained in a fraction not collected in the step (c);
(e) refolding the unfolded glycoprotein;
(f) fractionating the refolded glycoprotein by column chromatography and collecting a fraction having a predetermined activity; and
(g) repeating the steps (d) to (f) as needed.

The present invention further provides
a method for screening for a glycoprotein having a predetermined physiological activity, comprising the following steps (i) to (iii):
(i) folding a glycoprotein having uniform amino acid sequence and sugar chain;
(ii) fractionating the folded glycoprotein by column chromatography; and
(iii) measuring an activity of each of the fractions to determine whether or not it has a predetermined activity.

The present invention further provides
a method for obtaining a glycoprotein mixture having a desired physiological activity, comprising the following steps (A) to (D):
(A) folding a glycoprotein having uniform amino acid sequence and sugar chain;
(B) fractionating the folded glycoprotein by column chromatography;
(C) measuring an activity of each of the fractions; and
(D) determining a mixing ratio of the fractions to obtain a desired activity and mixing the fractions according to the ratio thus obtained.

Preferably, according to the production method of a glycoprotein, the screening method of a glycoprotein, or the method for obtaining a glycoprotein mixture having a desired physiological activity of the present invention, at least a part of the glycoprotein having uniform amino acid sequence and sugar chain are produced by a method comprising the following steps (1) to (6):
(1) esterifying a hydroxyl group of a resin having a hydroxyl group and a carboxyl group of an amino acid having an amino group protected with a fat-soluble protecting group or a carboxyl group of a glycosylated amino acid having an amino group protected with a fat-soluble protecting group;
(2) removing the fat-soluble protecting group to generate a free amino group;
(3) amidating the free amino group and a carboxyl group of an amino acid having an amino group protected with a fat-soluble protecting group or a carboxyl group of a glycosylated amino acid having an amino group protected with a fat-soluble protecting group;
(4) after the step (3), removing the fat-soluble protecting group to generate a free amino group;
(5) repeating the steps (3) and (4) once or more; and
(6) cleaving an ester bond formed in the step (1) by an acid.

Also, preferably, according to the above production method of a glycoprotein having uniform amino acid sequence and sugar chain,
a part of the glycoprotein having uniform amino acid sequence and sugar chain are produced by the steps (1) to (6), and the glycoprotein are produced by the method further comprising the following step (7):
(7) linking a part of the glycoprotein obtained in the step (6) with other peptides or glycopeptides by a ligation method.

### [Effects of the Invention]

According to the production method of a glycoprotein of the present invention, a glycoprotein having not only a uniform amino acid sequence and sugar chain structure but also a uniform higher order structure can be obtained. Thus, a glycoprotein uniformly exhibiting a predetermined physiological activity in addition to constant blood half-life and intracellular transportation can be produced.

Also, according to the method for screening for a glycoprotein of the present invention, a glycoprotein uniformly having a predetermined physiological activity can be selected from among a group of a glycoprotein exhibiting varied physiological activities due to different higher order structures. Because this glycoprotein has a uniform sugar chain structure, it also has a uniform sugar chain-based function such as the blood half-life and the intracellular transportation.

Also, according to the present invention, a glycoprotein mixture can be controlled so as to attain a desired activity.

The effects of the present invention as described above are advantageous particularly when a glycoprotein is used as a pharmaceutical product.

### [Brief Description of Drawings]

[Figure 1] Figure 1 shows the third domain of silver pheasant ovomucoid (OMSVP3) and the amino acid sequences of fragments 1 to 3, which are used for chemical synthesis of OMSVP3.
[Figure 2] Figure 2 shows Fragment 1 (thioesterified), which is used for chemical synthesis of OMSVP3.
[Figure 3] Figure 3 shows Fragment 2 (thioesterified), which is used for chemical synthesis of glycosylated OMSVP3.
[Figure 4] Figure 4 shows Fragment 3, which is used for chemical synthesis of OMSVP3.
[Figure 5] Figure 5 is a chromatogram at a wavelength of 220 nm at each stage of the synthesis of Fragment 1.
[Figure 6] Figure 6 is a chromatogram at a wavelength of 220 nm at each stage of the synthesis of Fragment 2.
[Figure 7] Figure 7 is a chromatogram at a wavelength of 220 nm at each stage of the synthesis of Fragment 3.
[Figure 8] Figure 8 is a chromatogram at a wavelength of 220 nm at each stage of linking of fragments 2 and 3 by NCL.
[Figure 9] Figure 9 is a chromatogram at a wavelength of 220 nm at each stage of linking of fragments 2 and 3 and Fragment 1 by NCL.
[Figure 10] Figure 10 is a chromatogram at a wavelength of 220 nm in separation of folded glycosylated OMSVP3 by HPLC.
[Figure 11] Figure 11 is a NMR spectrum of Fraction B in Figure 10.
[Figure 12] Figure 12 is a CD spectrum of Fraction B in Figure 10.
[Figure 13] Figure 13 shows the measurement results of the inhibitory activity of each of fractions in Figure 10 against chymotrypsin.
[Figure 14] Figure 14 shows Fragment 2' (thioesterified), which is used for chemical synthesis of non-glycosylated OMSVP3.
[Figure 15] Figure 15 is a chromatogram at a wavelength of 220 nm at each stage of the synthesis of Fragment 2'.
[Figure 16] Figure 16 is a chromatogram at a wavelength of 220 nm at each stage of linking of Fragment 2' and Fragment 3 by NCL.
[Figure 17] Figure 17 is a chromatogram at a wavelength of 220 nm at each stage of linking of fragments 2' and 3 and Fragment 1 by NCL.
[Figure 18] Figure 18 is a chromatogram at a wavelength of 220 nm in separation of folded non-glycosylated OMSVP3 by HPLC.
[Figure 19] Figure 19 is a NMR spectrum of Fraction F in Figure 18.
[Figure 20] Figure 20 is a CD spectrum of Fraction F in Figure 18.
[Figure 21] Figure 21 shows the measurement results of the inhibitory activity of each of fractions in Figure 18 against chymotrypsin.
[Figure 22] Figure 22 shows a calibration curve of Fraction F.
[Figure 23] Figure 23 shows the percent inhibition of Fractions A to D against chymotrypsin.
[Figure 24] Figure 24 shows the IC₅₀ values of Fractions A to D.
[Figure 25] Figure 25 shows the percent inhibition of Fractions E to H againsst chymotrypsin.
[Figure 26] Figure 26 shows the IC₅₀ values of Fractions E to H.
[Figure 27] Figure 27 shows a CD spectrum of Fraction B in various temperatures.
[Figure 28] Figure 28 shows a CD spectrum of Fraction F in various temperatures.
[Figure 29] Figure 29 is a chromatogram at a wavelength of 220 nm in thermolysin digestion of Fraction B.
[Figure 30] Figure 30 shows the results of mass spectrometric analysis of peptide fragments resulting from thermolysin digestion of Fraction B.
[Figure 31] Figure 31 is a chromatogram at a wavelength of 220 nm in thermolysin digestion of Fraction F.
[Figure 32] Figure 32 shows the results of mass spectrometric analysis of peptide fragments resulting from thermolysin digestion of Fraction F.
[Figure 33] Figure 33 shows the results of determination of the position of a disulfide bond by thermolysin digestion of Fraction B.
[Figure 34] Figure 34 shows the results of determination of the position of a disulfide bond by thermolysin digestion of Fraction F.
[Figure 35] Figure 35 shows the calibration curve of the substrate peptide.
[Figure 36] Figure 36 shows the Michaelis-Menten plot of the substrate peptide.
[Figure 37] Figure 37 shows the reaction rate of the substrate peptide per unit time.

### [Detailed Description of the Preferred Embodiments]

Hereinbelow, preferred embodiments of the present invention will be described.

As used herein, a "protein" is not particularly limited as long as it is an assembly of a plurality of amino acids bound by an amide bond, and includes a known protein, a novel protein, and a modified protein. In a preferred embodiment, in the protein moiety of the glycoprotein obtained by the production method of the present invention, a plurality of amino acids are bound by the same amide bond as a naturally-occurring protein (peptide bond). The protein as used herein has enough length to be folded into a predetermined higher order structure.

As used herein, a "modified protein" refers to a naturally or artificially modified protein. Examples of such modification include alkylation, acylation (for example, acetylation), amidation (for example, amidation of the C-terminus of a protein), carboxylation, formation of an ester, formation of a disulfide bond, glycosylation, lipidation, phosphorylation, hydroxylation, and binding of a labeling compound, which are applied to one or more amino acid residues of a protein.

The term "peptide" as used herein is used as a synonym for protein in principle. However, it may also be used to refer a part of a protein and a relatively short amino acid chain which does not form a higher order structure.

As used herein, an "amino acid" is used in the broadest sense, and examples thereof include, in addition to naturally-occurring amino acid such as serine (Ser), asparagine (Asn), valine (Val), leucine (Leu), isoleucine (Ile), alanine (Ala), tyrosine (Tyr), glycine (Gly), lysine (Lys), arginine (Arg), histidine (His), aspartic acid (Asp), glutamic acid (Glu), glutamine (Gln), threonine (Thr), cysteine (Cys), methionine (Met), phenylalanine (Phe), tryptophan (Trp), and proline (Pro), a
non-naturally-occurring amino acid such as a mutant and a derivative of an amino acid. Those skilled in the art would understand in consideration of the above broad definition that examples of an amino acid used in the present invention include an L-amino acid; a D-amino acid; a
chemically-modified amino acid such as a mutant and a derivative of an amino acid; a non-protein constituent amino acid in the living body such as norleucine, β-alanine, and ornithine; and a chemically-synthesized compound having characteristics of an amino acid that is known to those skilled in the art. Examples of a non-naturally-occurring amino acid include a α-methylamino acid (for example, α-methylalanine), a D-amino acid, a histidine-like amino acid (for example, 2-amino-histidine, β-hydroxyl-histidine, homohistidine, α-fluoromethyl-histidine, and
α-methyl-histidine), an amino acid having an extra methylene in the side chain (a "homo" amino acid), and an amino acid in which a carboxylic acid functional group in the side chain is replaced with a sulfonic acid group (for example, a cysteic acid).

In a preferred embodiment, the protein moiety of the glycoprotein obtained by the production method of the present invention is entirely composed of amino acids that are present in the living body as constituent amino acids of a protein or a glycoprotein.

As used herein, a "glycoprotein" is not particularly limited as long as it is a compound obtained by adding at least one sugar chain to the aforementioned protein, and includes a known glycoprotein and a novel glycoprotein. The term "glycopeptide" as used herein is used as a synonym for glycoprotein in principle. However, it may also be used to indicate a part of a glycoprotein and a peptide obtained by binding a sugar chain to the aforementioned peptide.

In a preferred embodiment, the glycoprotein obtained by the production method of the present invention is a protein having a N-linked sugar chain or an O-linked sugar chain, and examples thereof include a part or all of a peptide such as erythropoietin, interleukin, interferon-β, an antibody, monocyte chemotactic protein-3 (MCP-3), and an ovomucoid protein.

In a glycoprotein, a sugar chain and an amino acid residue of the protein may be bound directly or via a linker. Although no particular limitation is imposed on the binding site of the sugar chain and the amino acid, an amino acid is preferably bound to the reducing end of the sugar chain.

No particular limitation is imposed on the kind of amino acid to which a sugar chain is bound, and a sugar chain may be bound to either a naturally occurring or non-naturally occurring amino acid. From the viewpoint that the glycoprotein has the same or similar structure to a glycoprotein present in the living body, the sugar chain is preferably bound to Asn as a N-linked sugar chain or to Ser or Thr as an O-linked sugar chain. Particularly, in the case of a N-linked sugar chain, the glycoprotein obtained by the production method of the present invention is preferably a glycoprotein having a structure in which a sugar chain is bound to Asn, and an amino acid (X) other than proline is bound to the C-terminus of the Asn by an amide bond (peptide bond), and further, Thr or Ser is bound to the C-terminus of the X by an amide bond (peptide bond) (-glycosylated Asn-X-Thr/Ser-). When the sugar chain and the amino acid are bound via a linker, from the viewpoint of a property of easy binding to the linker, the amino acid to which a sugar chain is bound is preferably an amino acid having two or more carboxyl groups in its molecule such as aspartic acid and glutamic acid; an amino acid having two or more amino groups in its molecule such as lysine, arginine, histidine, and tryptophan; an amino acid having a hydroxyl group in its molecule such as serine, threonine, and tyrosine; an amino acid having a thiol group in its molecule such as cysteine; or an amino acid having an amide group in its molecule such as asparagine and glutamine. Particularly, from the viewpoint of reactivity, aspartic acid, glutamic acid, lysine, arginine, serine, threonine, cysteine, asparagine, or glutamine is preferable.

When a sugar chain and an amino acid are bound via a linker in a glycoprotein, substances used in the art can be widely used as the linker, and examples thereof include:

-NH- (CO)-(CH₂)ₐ-CH₂-

wherein, a is an integer, and although no particular limitation is imposed thereon as long as it does not block the intended function of the linker, it is preferably an integer of 0 to 4;
C₁₋₁₀ polymethylene; and

-CH₂-R³-

wherein, R³ is a group produced by removing one hydrogen atom from a group selected from the group consisting of alkyl, substituted alkyl, alkenyl, substituted alkenyl, alkynyl, substituted alkynyl, aryl, substituted aryl, a cyclic carbon group, a substituted cyclic carbon group, a heterocyclic group, and a substituted heterocyclic group.

As used herein, a "sugar chain" encompasses, in addition to a compound consisting of a chain of two or more unit sugars (monosaccharide and/or a derivative thereof), a compound consisting of single unit sugar (monosaccharide and/or a derivative thereof). Examples of such a sugar chain widely include, but are not limited to, monosaccharides and polysaccharides contained in the living body (glucose, galactose, mannose, fucose, xylose, N-acetylglucosamine, N-acetylgalactosamine, sialic acid, as well as a complex and a derivative of these monosaccharides and polysaccharides), and further, a decomposed polysaccharide, a glycoprotein, proteoglycan, glycosaminoglycan, and a sugar chain decomposed or derived from a complex biological molecule such as a glycolipid. When two or more unit sugars are linked in a chain, the unit sugars are bound to each other via dehydration condensation of the glycoside bond. The sugar chain may be linear or branched.

Also, as used herein, a "sugar chain" encompasses a derivative of a sugar chain. Examples of a derivative of a sugar chain include, but are not limited to, a sugar chain having, as the constituent sugar of the sugar chain, a sugar having a carboxyl group (for example, aldonic acid that is converted into carboxylic acid through oxidation of the C-1 position (for example, D-gluconic acid resulting from oxidation of D-glucose), uronic acid having its terminal C atom converted into a carboxylic acid (for example, D-glucuronic acid resulting from oxidation of D-glucose), a sugar having an amino group or a derivative of an amino group (for example, an acetylated amino group) (for example, N-acetyl-D-glucosamine and N-acetyl-D-galactosamine), a sugar having both an amino group and a carboxyl group (for example, N-acetylneuraminic acid (sialic acid) and N-acetylmuramic acid), a deoxylated sugar (for example, 2-deoxy-D-ribose), a sulfated sugar containing a sulfuric acid group, and a phosphorylated sugar containing a phosphate group.

The sugar chain of the present invention is preferably a sugar chain that is present as a complex sugar in the living body (such as a glycoprotein (or a glycopeptide), a proteoglycan, and a glycolipid), and preferably a N-linked sugar chain, an O-linked sugar chain, and the like, which are a sugar chain bound to a protein (or a peptide) to form a glycoprotein (or a glycopeptide) in the living body. In a glycoprotein having an O-linked sugar chain, N-acetylgalactosamine (GalNAc), N-acetylglucosamine (GlcNAc), xylose, fucose, and the like are bound to Ser or Thr of a peptide through an O-glycosidic bond, and a sugar chain is further added thereto. Examples of an N-linked sugar chain include a high-mannose-type, a complex-type, and a hybrid-type, among which a complex-type is preferable.

In the present invention, an example of a preferable sugar chain is one represented by the following formula (4). 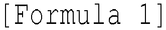 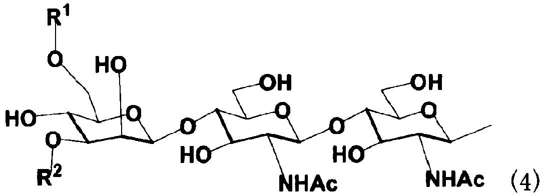 wherein, R¹ and R² are each independently a hydrogen atom or a group represented by the formulas (5) to (8).

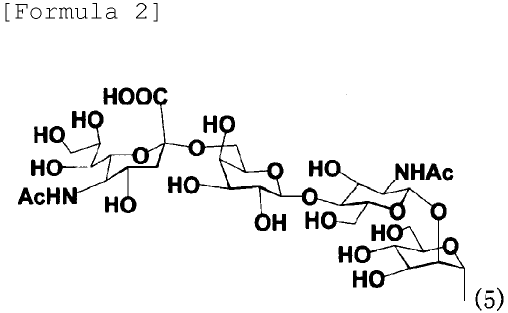

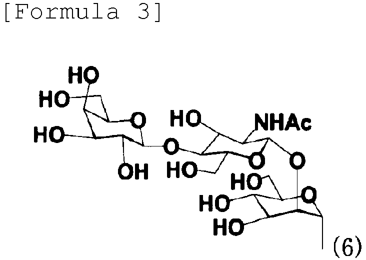

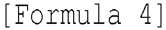

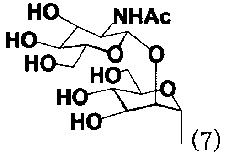

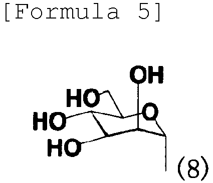

When considering applying the production method of a glycoprotein of the present invention to the field of the production of a pharmaceutical product and the like, from the viewpoint of possible avoidance of a problem of antigenicity and the like, examples of a preferable sugar chain include a sugar chain having the same structure as a sugar chain that is bound to a protein and present as a glycoprotein in the human body (for example, the sugar chain described in FEBS LETTERS Vol. 50, No. 3, Feb. 1975) (a sugar chain having the same kind of constituent sugars and the same binding pattern of these constituent sugars) or a sugar chain obtained by eliminating one or more sugars from the non-reducing end of the above sugar chain.

No particular limitation is imposed on the number of sugar chains to be added in a glycoprotein as long as it is one or more; however, from the viewpoint of the provision of a glycoprotein having a similar structure to a glycoprotein present in the living body, the number of sugar chains to be added might be more preferable if it is approximately the same number as a glycoprotein present in the body.

In the production method of a glycoprotein of the present invention, a glycoprotein having uniform amino acid sequence and sugar chain is used. In the present invention, the structure of the sugar chain in a glycoprotein being uniform means that, when glycoproteins are compared among them, the sugar chain addition site in a peptide, the kind of each constituent sugar of the sugar chain, the binding order, and the binding pattern of sugars are the same in at least 90% or more, preferably 95% or more, more preferably 99% or more of the sugar chain.

Also, in the present invention, the amino acid sequence in a glycoprotein being uniform means that, when glycoproteins are compared among them, the kind of amino acid in the protein, the binding order, and the binding pattern of amino acids are the same. However, as long as a folded glycoprotein has a predetermined activity, the above-noted properties may be the same in at least 90% or more, preferably 95% or more, more preferably 99% or more of the glycoprotein.

The glycoprotein having uniform amino acid sequence and sugar chain to be used in the present invention can be produced by incorporating a step of adding a sugar chain into a production method of a peptide known to those skilled in the art such as solid phase synthesis, liquid-phase synthesis, synthesis by cells, and a method of separating and extracting a naturally-occurring one. Concerning the production method of a sugar chain to be used in the step of adding a sugar chain, for example International Publication Nos. WO03/008431, WO2004/058984, WO2004/008431, WO2004/058824, WO2004/070046, WO2007/011055, and the like can be referred to.

In a preferred embodiment of the present invention, at least a part of the glycoprotein having uniform amino acid and sugar chain are produced by the following method. The pamphlet of WO2004/005330 can also be referred to for the method shown below.

Firstly, (1) a hydroxyl group of a resin having a hydroxyl group is esterified with a carboxyl group of an amino acid having an amino group protected with a fat-soluble protecting group or a carboxyl group of a glycosylated amino acid having an amino group protected with a fat-soluble protecting group. In that case, because the amino group of an amino acid is protected with a fat-soluble protecting group, the self-condensation of amino acid is prevented and the esterification reaction will occur between the hydroxyl group of a resin and the carboxyl group of an amino acid.

Then, (2) the fat-soluble protecting group of the ester produced in the step (1) is removed to generate a free amino group,
(3) the aforementioned free amino group is amidated with a carboxyl group of an amino acid having an amino group protected with a fat-soluble protecting group or a carboxyl group of a glycosylated amino acid having an amino group protected with a fat-soluble protecting group,
(4) after the step (3), the fat-soluble protecting group is removed to generate a free amino group, and
(5) the steps (3) and (4) are repeated one or more times as needed, whereby a glycoprotein having a desired number of amino acids linked together and having one or more sugar chains bound to a desired position can be obtained. Examples of a glycosylated amino acid include an asparagine-linked sugar chain in which a sugar chain is bound to nitrogen of an amide group in the side chain of asparagine by a N-glycoside bond and a serine-linked sugar chain or a threonine-linked sugar chain in which a sugar chain is bound to a hydroxyl group of the side chain of serine or threonine by an O-glycoside bond.

The glycoprotein obtained by the step (5) is bound to resin at one end, while having a free amino group at the other end. Thus, (6) a desired glycoprotein can be produced by cleaving an ester bond formed in the step (1) by an acid.

As solid-phase resin, resin generally used in solid phase synthesis may be employed, and examples thereof include Amino-PEGA resin (the product of Merck), Wang resin (the product of Merck), HMPA-PEGA resin (the product of Merck), and Trt Chloride resin (the product of Merck).

Also, a linker can be present between Amino-PEGA resin and an amino acid, and examples of such a linker include 4-hydroxymethylphenoxyacetic acid (HMPA) and 4-(4-hydroxymethyl-3-methoxyphenoxy)-butylacetic acid (HMPB).

Examples of a fat-soluble protecting group include, but are not particularly limited to, a protecting group such as a group containing a carbonyl group such as a 9-fluorenylmethoxycarbonyl (Fmoc) group, a t-butyloxycarbonyl (Boc) group, and an allyloxycarbonyl (Alloc) group, an acyl group such as an acetyl (Ac) group, an allyl group, and a benzyl group.

In order to introduce a fat-soluble protecting group, for example when introducing a Fmoc group, it can be introduced by carrying out reactions with the addition of 9-fluorenylmethyl-N-succinimidyl carbonate and sodium hydrogen carbonate. The above reaction may be carried out at 0 to 50°C, preferably room temperature, for approximately one to five hours.

As an amino acid protected with a fat-soluble protecting group, one obtained by protecting the aforementioned amino acid by the method described as above can be used. Further, a commercially available amino acid can also be used. Examples thereof include Fmoc-Ser, Fmoc-Asn, Fmoc-Val, Fmoc-Leu, Fmoc-Ile, Fmoc-Ala, Fmoc-Tyr, Fmoc-Gly, Fmoc-Lys, Fmoc-Arg, Fmoc-His, Fmoc-Asp, Fmoc-Glu, Fmoc-Gln, Fmoc-Thr, Fmoc-Cys, Fmoc-Met, Fmoc-Phe, Fmoc-Trp, and Fmoc-Pro.

As an esterifying catalyst, a known dehydration condensing agent such as 1-mesitylenesulfonyl-3-nitro-1,2,4-triazole (MSNT), dicyclohexylcarbodiimide (DCC), and 1,3-diisopropylcarbodiimide (DIPCDI) can be used. With regard to the proportion of an amino acid and a dehydration condensing agent used, relative to one part by weight of the former, the latter is normally one to 10 parts by weight, preferably two to five parts by weight.

Preferably, an esterification reaction is carried out by, for example, placing resin in a solid phase column and washing the resin with a solvent, followed by addition of an amino acid solution. Examples of a solvent for washing include dimethylformamide (DMF), 2-propanol, and methylene chloride. Examples of a solvent for dissolving an amino acid include dimethyl sulfoxide (DMSO), DMF, and methylene chloride. The esterification reaction may be carried out at 0 to 50°C, preferably room temperature, for approximately 10 minutes to 30 hours, preferably 15 minutes to 24 hours.

At this time, it is also preferable to cap any unreacted functional group on the solid phase by acetylating with anhydrous acetic acid and the like.

Removal of a fat-soluble protecting group can be carried out by, for example, treatment with a base. Examples of a base include piperidine and morpholine. At this time, the reaction is preferably carried out in the presence of a solvent. Examples of a solvent include DMSO, DMF, and methanol.

An amidation reaction of a free amino group and a carboxyl group of any amino acid in which nitrogen of the amino group is protected with a fat-soluble protecting group is preferably carried out in the presence of an activator and a solvent.

Examples of an activator include dicyclohexylcarbodiimide (DCC), 1-ethyl-3-(3-dimethylaminopropyl)carbodiimide hydrochloride (WSC/HCl), diphenylphosphoryl azide (DPPA), carbonyldiimidazole (CDI), diethyl cyanophosphonate (DEPC), 1,3-diisopropylcarbodiimide (DIPCI), benzotriazole-1-yl-oxy-tris-pyrrolidinophosphonium hexafluorophosphate (PyBOP), 3-diethoxyphosphoryloxy-1,2,3-benzotriazin-4(3H)-one (DEPBT), 1-hydroxybenzotriazole (HOBt), hydroxysuccinimide (HOSu), dimethylaminopyridine (DMAP), 1-hydroxy-7-azabenzotriazole (HOAt), 3-hydroxy-4-oxo-3,4-dihydro-5-azabenzo-1,2,3-triazine (HODhbt), hydroxyphthalimide (HOPht), pentafluorophenol (Pfp-OH), 2-(1H-benzotriazole-1-yl)-1,1,3,3-tetramethyluronium hexafluorophosphate (HBTU), O-(7-azabenzotriazol-1-yl)-1,1,3,3-tetramethyluronium hexafluorophosphonate (HATU), and O-benzotriazol-1-yl-1,1,3,3-tetramethyluronium tetrafluoroborate (TBTU).

The activator is preferably used in an amount of one to 20 equivalents, preferably one to 10 equivalents, more preferably one to five equivalents relative to any amino acid in which nitrogen of the amino group is protected with a fat-soluble protecting group.

Although reactions proceed using only the aforementioned activators, amine is preferably used in combination as a supplemental agent. Examples of amine include diisopropylethylamine (DIPEA), N-ethylmorpholine (NEM), N-methylmorpholine (NMM), and N-methylimidazole (NMI). The supplemental agent is preferably used in an amount of one to 20 equivalents, preferably one to 10 equivalents, more preferably one to five equivalents relative to any amino acid in which nitrogen of the amino group is protected with a fat-soluble protecting group.

Examples of a solvent include DMSO, DMF, and methylene chloride. The reaction may be carried out at 0 to 50°C, preferably room temperature, for approximately 10 minutes to 30 hours, preferably approximately 15 minutes to 24 hours. Also at this time, it is preferable to cap any unreacted amino group on the solid phase by acetylating with anhydrous acetic acid and the like. The fat-soluble protecting group can be removed in a similar manner as above.

Cleavage of a peptide chain from resin is preferably processed with an acid. Examples of an acid include trifluoroacetic acid (TFA) and hydrogen fluoride (HF). At this time, a linker between the fat-soluble protecting group in an amino acid and resin may produce a highly reactive cationic species. Thus, in order to capture such a cationic species, a nucleophilic reagent is preferably added. Examples of a nucleophilic reagent include triisopropylsilane (TIS), phenol, thioanisole, and ethanediol (EDT).

A glycoprotein having uniform amino acid sequence and sugar chain may be produced as follows; dividing it into several peptide blocks or glycopeptide blocks and synthesizing each block by the steps (1) to (6), and then linking the blocks thus synthesized together by the ligation method.

As used herein, the "ligation method" encompasses Native Chemical Ligation (NCL) as described in International Publication No. WO96/34878, and it also encompasses application of the Native Chemical Ligation to a peptide including non-naturally-occurring amino acids and amino acid derivatives. According to the ligation method, a protein having a natural amide bond (peptide bond) at the binding site can be produced.

Linking by ligation can be applied to link between any of peptide-peptide, peptide-glycopeptide, and glycopeptide-glycopeptide; however, it is necessary that one of two peptides or glycopeptides to be linked has a cysteine residue at its N-terminus and the other has a α-carboxythioester moiety at its C-terminus.

In order for each peptide or glycopeptides to have a cysteine residue at its N-terminus, for example, when designing each peptide or glycopeptide block, the division may be made in the N-terminal side of a cysteine residue contained in a glycoprotein to be produced as a final product.

A peptide or glycopeptide having a α-carboxythioester moiety at its C-terminus can be produced by the method known to those skilled in the art such as the method described in International Publication No. WO96/34878.

For example, as will be described in Examples later, a protected peptide (or glycopeptide) in which the amino acid side chain and the N-terminal amino acid are protected is obtained by the solid phase synthesis method, and the carboxyl group at the C-terminal of this protected peptide (or glycopeptide) is condensed with benzyl mercaptan in a liquid phase, using benzotriazole-1-yl-oxy-tris-pyrrolidino-phosphonium hexafluorophosphate (PyBOP)/DIPEA as a condensing agent, and then the resulting peptide (or glycopeptides) is deprotected using a 95% TFA solution, whereby a peptide (or glycopeptide) having a α-carboxythioester at its C-terminus can be obtained.

The ligation method can be carried out by the method known to those skilled in the art such as the method described in Patent Literature 1, or referring to the description of Examples to be presented later. For example, a first peptide having a α-carboxythioester moiety represented by -C(=O) -SR at its C-terminus and a second peptide having an amino acid residue having a -SH group at its N-terminus are prepared in reference to the aforementioned description. Although no particularly limitation is imposed on R in the first peptide as long as it does not block a thiol exchange reaction and becomes a leaving group in a nucleophilic substitution reaction performed on the carbonyl group, it is preferably selected from a benzyl-type such as benzyl mercaptan, an aryl-type such as thiophenol, 4-(carboxymethyl)-thiophenol, an alkyl-type such as 2-mercaptoethanesulfonate and 3-mercaptopropionamide, and the like. Also, although the -SH group at the N-terminus of the second peptide may be protected with a protecting group as desired, this protecting group is removed at a desired point in the reaction before it proceeds to the below-described ligation reaction, and the second peptide having a -SH group at its N-terminus reacts with the first peptide. For example when a protecting group is one that will be spontaneously removed in the conditions in which ligation occurs, such as a disulfide group, the second peptide protected with a protecting group can be used as-is in the following ligation reaction.

These two peptides are mixed in a solution such as a 100 mM phosphate buffer in the presence of catalytic thiol such as 4-mercaptophenyl acetic acid, benzyl mercaptan, and thiophenol. Preferably, the reaction is carried out in the proportion of 0.5 to two equivalents of the second peptide and five equivalents of catalytic thiol relative to one equivalent of the first peptide. The reaction is preferably carried out in the conditions of a pH of approximately 6.5 to 7.5 and a temperature of approximately 20 to 40°C for approximately one to 30 hours. The progress of the reaction can be confirmed by a known technique of a combination of HPLC, MS, and the like.

To the above reaction, a reducing agent such as dithiothreitol (DTT) and tris2-carboxyethylphosphine hydrochloride (TCEP) is added to suppress a side reaction, and the resulting product is subjected to purification if desired, whereby the first peptide and the second peptide can be linked.

When the peptide having a carboxythioester moiety (-C=O-SR) at its C-terminus has different R groups, the order of the ligation reaction can be manipulated (refer to Protein Science (2007), 16: 2056-2064, and the like), which can be taken into consideration when the ligation is carried out multiple times. For example, when an aryl group, a benzyl group, and an alkyl group are present as R, the ligation reaction generally proceeds in this order.

As used herein, the "higher order structure" of a protein refers to a conformation of a protein encompassing the secondary structure such as a α-helix and a β-sheet structure or a structure such as a random coil, the tertiary structure in which the secondary structure is spatially folded by a hydrogen bond, a disulfide bond, an ionic bond, a hydrophobic interaction, and the like so as to form a stable conformation, and the quaternary structure which is formed by assembling a plurality of polypeptide chains as subunits. The higher order structure of a protein is preferably a structure necessary for the protein to exhibit its function in the living body. The higher order structure of a protein can be analyzed by X-ray crystallography, NMR, and the like.

As used herein, glycoprotein having uniform higher order structure means that, when comparing among the glycoprotein, the higher order structure of the protein moiety of the glycoprotein is substantially the same. The higher order structure being substantially the same means that at least 90% or more, preferably 95% or more, more preferably 99% or more of the structure are uniform. The glycoprotein having uniform higher order structure has stable quality, and thus is preferable particularly in a field such as the production of pharmaceutical product and the assay. Whether or not the high order structure of a glycoprotein contained in an arbitrary fraction is uniform or not can be confirmed by, for example, a NMR analysis, a CD measurement, and disulfide mapping.

As used herein, the "folding" means that the protein moiety of a glycoprotein is folded into a specific higher order structure. While those skilled in the art can appropriately carry out the folding of a glycoprotein by a known method or an equivalent method, examples of such a method include the dialysis method, the dilution method, and the inactivation method. The dialysis method is a method for folding a peptide into a predetermined higher order structure in which a protein denaturing agent (unfolding agent) is added in advance, after which the resulting mixture is gradually diluted by dialysis so as to be replaced by a buffer and the like. Examples of an unfolding agent include guanidine hydrochloride and urea. Also, the dilution method is a method for folding a peptide into a higher order structure in which, after addition of a protein denaturing agent, the resulting mixture is diluted by a buffer and the like in a stepwise manner or at once. The inactivation method is a method for folding a peptide into a higher order structure in which, after addition of a protein denaturing agent, a second agent inactivating the denaturing agent is added in a stepwise manner or at once.

In the present invention, the "predetermined physiological activity" can be selected from among physiological activities of a glycoprotein having a higher order structure that is obtained with good reproducibility at a constant ratio when folded. Such a physiological activity can be obtained by folding a target glycoprotein in advance by a method similar to the steps (a) and (b) to be described later and fractionating it by column chromatography, and collecting the eluent corresponding to the major peak, and then measuring the physiological activity of the glycoprotein contained in that fraction. Here, the major peak means a peak obtained with good reproducibility when the steps (a) and (b) are performed repeatedly. The physiological activity can be measured by a method known to those skilled in the art depending on the target glycoprotein.

In the "method for producing a glycoprotein having uniform amino acid sequence, sugar chain structure, and higher order structure" according to the present invention, firstly in the step (a), a glycoprotein having uniform amino acid sequence and sugar chain is folded. In the solution containing the folded glycoprotein, a mixture of glycoproteins with different higher order structures is present, containing ones with or without a predetermined activity.

Then, in the step (b), the folded glycoprotein is fractionated by column chromatography. Although no particularly limitation is imposed on the column chromatography as long as it can separate a glycoprotein having different higher order structure, for example high-performance liquid chromatography (HPLC) can be used. While those skilled in the art can appropriately select the conditions such as the kind of the solid phase and the mobile phase and the outflow rate of column chromatography according to the glycoprotein to be separated, for example ODS-type reverse phase chromatography, normal phase chromatography, affinity column, gel filtration column, ion-exchange column, and the like can be used.

In the step (c), the activity of the glycoprotein contained in each of fractions of the eluate of the column chromatography is measured and a fraction having a predetermined activity is collected, whereby the glycoprotein having uniform amino acid sequence, sugar chain structure, and higher order structure can be obtained.

Alternatively, the glycoprotein production method of the present invention preferably includes, after the step (c), (d) unfolding glycoprotein contained in a fraction not collected in the aforementioned step (c),
(e) refolding the unfolded glycoprotein;
(f) fractionating the refolded glycoprotein by column chromatography and collect afraction having the aforementioned desired activity; and
(g) repeating the steps (d) to (f) as needed.

The fraction containing the glycoprotein to be unfolded in the step (d) also contains a higher order structure lacking a predetermined activity. Also, because a fraction containing a mixture of two or more kinds of glycoproteins having a predetermined activity does not exhibit a predetermined level of activity either, such a fraction is also included in the fraction to be subjected to unfolding.

Although the glycoprotein can be unfolded by a method known to those skilled in the art, examples of such a method include a method of adding an unfolding agent (protein denaturing agent) such as guanidine hydrochloride and urea and a method of adding, in addition to the above-noted agent, a reducing agent such as dithiothreitol (DTT) and mercaptoethanol.

The steps (e) and (f) can be carried out by a method similar to the aforementioned steps (a) to (c).

As described above, by carrying out the steps (d) to (f), the glycoprotein contained in a fraction lacking a predetermined activity is unfolded once and then refolded, whereby the glycoprotein may possibly be converted into a higher order structure having a predetermined activity at a constant ratio. In this way, the glycoprotein with a higher order structure having a predetermined activity can be maximally collected.

"A method for screening for a glycoprotein" according to the present invention includes (i) folding a glycoprotein having uniform amino acid sequence and sugar chain,
(ii) fractionating the folded glycoprotein by column chromatography, and
(iii) measuring an activity of each of the fractions to determine whether or not it has a predetermined activity.

The steps (i) and (ii) can be carried out similarly to the aforementioned steps (a) and (b). As described above, in the solution containing the folded glycoprotein having uniform amino acid sequence and sugar chain, a mixture of glycoproteins with various higher order structures is present. Accordingly, after fractionating by column chromatography, the activity of each of fractions is measured so as to determine whether or not it has a predetermined activity, whereby only the glycoprotein having uniform higher order structure and having a predetermined physiological activity can be selected and purified.

The present invention also provides a method for obtaining a glycoprotein mixture having a desired physiological activity. This method includes (A) folding a glycoprotein having uniform amino acid sequence and sugar chain,
(B) fractionating the folded glycoprotein by column chromatography,
(C) measuring an activity of each of the fractions, and
(D) determining a mixing ratio of the fractions with which a desired activity is obtainable and mixing the fractions according to the ratio thus obtained.

The steps (A) and (B) can be carried out similarly to the aforementioned steps (a) and (b). The steps (A) and (B) give a glycoprotein having uniform amino acid sequence, sugar chain structure, and higher order structure and having a predetermined activity. Accordingly, the glycoprotein can be mixed at a predetermined ratio to obtain a glycoprotein mixture having a predetermined activity.

The terms as used herein are presented in order to explain a specific embodiment without any intention to limit the invention.

Also, unless the context clearly requires otherwise, the terms "comprise, contain, include, or encompass" as used herein refer the presence of a described matter (a member, a step, a factor, a number, and the like) and these terms do not exclude the presence of other matters (a member, a step, a factor, a number, and the like).

Unless there is no alternative definition, all the terms used herein (including technical terms and scientific terms) have the same meaning as widely understood by those skilled in the field to which the present invention pertains. Unless an alternative definition is not clearly indicated, the terms used herein should be construed to have the meaning that is consistent with the meaning as in the present specification and a relevant technical field but should not be either idealized or construed in the excessively formalized sense.

The embodiment of the present invention may be explained while referring to a schematic diagram; however, in the case of a schematic diagram, the diagram may be presented in an exaggerated manner so as to clearly explain the invention.

Although the terms such as first and second are used to express various factors, it should be understood that these factors are not limited by such terms. These terms are used solely to distinguish one factor from another, and for example, it is possible to describe a first factor as a second factor, and similarly, to describe a second factor as a first factor without departing from the scope of the present invention.

Hereinbelow, the present invention will be described in more detail in reference to Examples.

### [Examples]

### <Example 1> Chemical synthesis of the third domain of silver pheasant ovomucoid (hereinafter, may be referred to as OMSVP3)

### 1. Chemical synthesis of the third domain of silver pheasant ovomucoid having uniform amino acid sequence and sugar chain

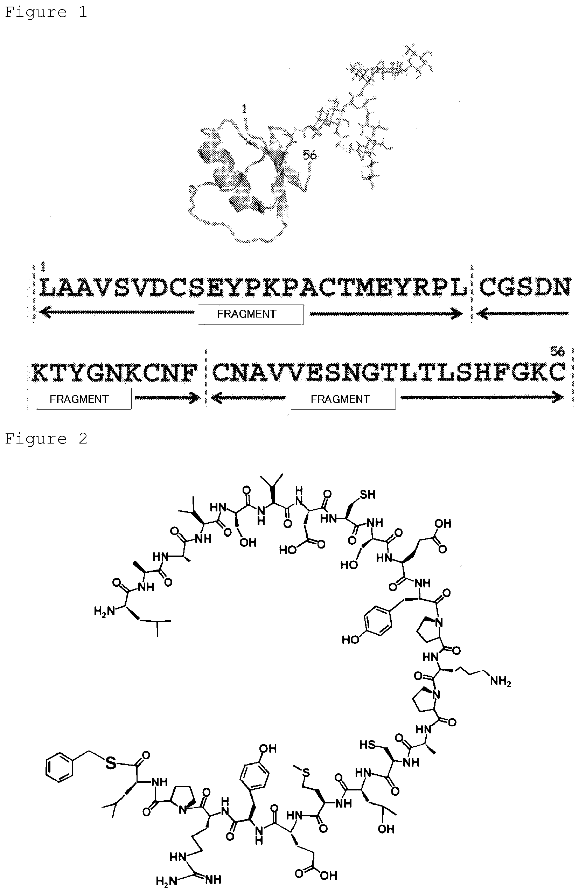

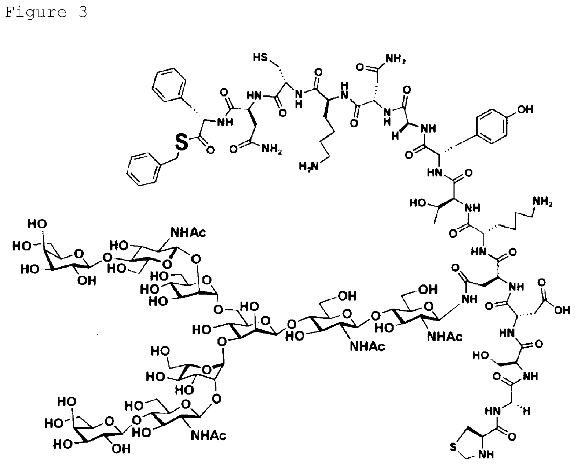

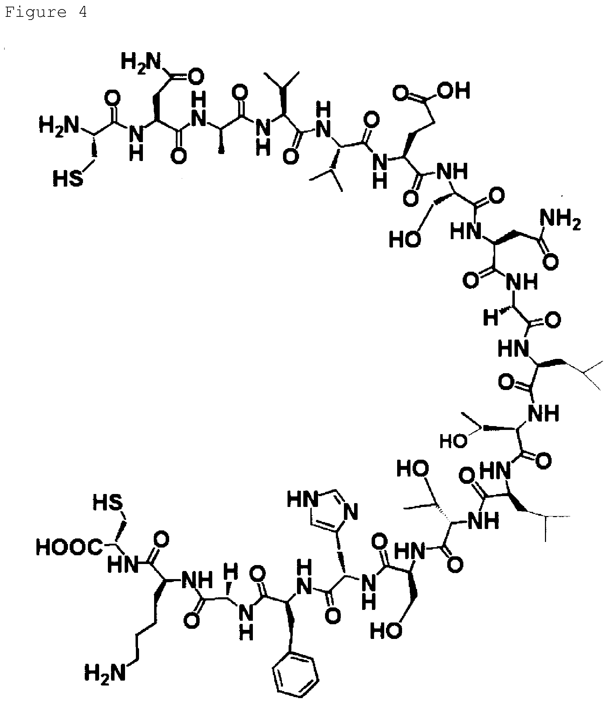

Three fragments as shown in Figure 1 were each synthesized and then ligated by NCL to synthesize a third domain of silver pheasant ovomucoid having uniform amino acid sequence and sugar chain. Fragments 1 to 3 are shown in Figures 2 to 4.

### [Instruments used]

¹H-NMR was measured by AVANCE 600 (shown as 600 MHz) of Bruker Corporation. For the ESI mass spectrum measurement, Esquire 3000 plus. of Brucker Daltonics Corporation was used.

For the CD spectrum measurement, J-820 and J-805 of JASCO Corporation were used.

As a RP-HPLC analytical instrument, one manufactured by Waters Corporation, and as a UV detector, Waters 486, a photodiode array detector (Waters 2996), and Waters 2487, all were manufactured by Waters Corporation, and as a column, Cadenza column (Imtakt Corp., 3 µm, 4.6 × 75 mm), VydacC-18 (5 µm, 4.6 × 250 mm, 10 × 250 mm), Vydac-8 (5 µm, 10 × 250 mm), and VydacC-4 (5 µm, 4.6 × 250 mm), were used.

### [Synthesis of Fragment 1]

Into a solid phase synthesis column, 2-chlorotrityl resin (143 mg, 200 µmol) was placed, which was then sufficiently washed with methylene chloride (DCM). Separately, DCM (1.2 mL) having Fmoc-Leu (212.1 mg, 0.6 mmol) and DIPEA (272.1 µL, 1.6 mmol) dissolved therein was prepared, and poured into the solid phase synthesis column charged with the resin, followed by stirring at room temperature for two hours. After stirring, the resin was washed with DCM : MeOH : DIPEA = 17 : 2 : 1, DCM, and DMF. Subsequently, the Fmoc group was deprotected with a 20% piperidine/DMF solution (2 mL) for 20 minutes. The resulting product was washed with DMF and the reaction was confirmed with Kaiser Test. Thereafter, the peptide chain extension was carried out by sequentially condensing amino acids using the method shown below.

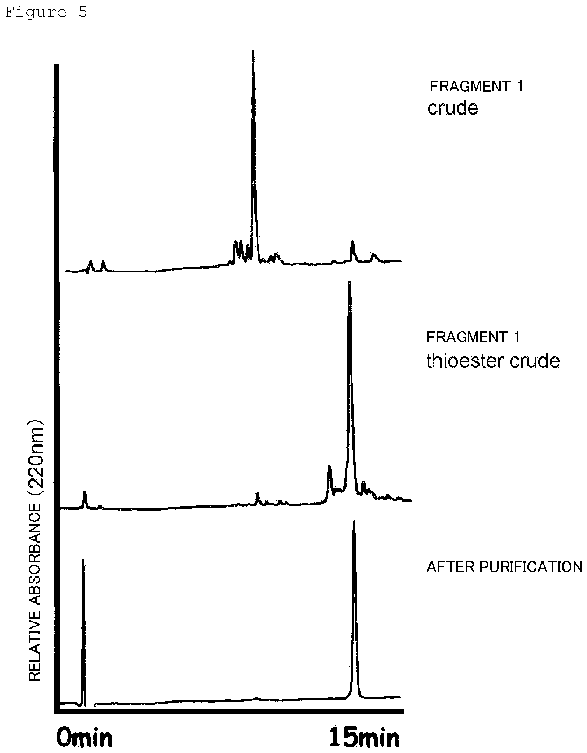

An amino acid having an amino group protected with a Fmoc group and HOBt (135.1 mg, 1 mmol), and DIPCI (153.9 µL, 1 mmol) were dissolved in DMF (4 mL) and the resulting solution was activated for 15 minutes. Thereafter, the solution was poured into the solid phase synthesis column, followed by stirring at room temperature for one hour. After stirring, the resin was washed with DCM and DMF. The Fmoc-group was deprotected with a 20% piperidine/DMF solution (2 mL) for 20 minutes. The above operation was repeated to sequentially condense amino acids. As the amino acid having a protected amino group, Fmoc-Pro, Fmoc-Arg(Pbf), Fmoc-Tyr(tBu), Fmoc-Glu(OtBu), Fmoc-Met, Fmoc-Thr(tBu), Fmoc-Cys(Trt), Fmoc-Ala, Fmoc-Pro, Fmoc-Lys(Boc), Fmoc-Pro, Fmoc-Tyr(tBu), Fmoc-Glu(OtBu), Fmoc-Ser(tBu), Fmoc-Cys(Trt), Fmoc-Asp(OtBu), Fmoc-Val, Fmoc-Ser(tBu), Fmoc-Val, Fmoc-Ala, and Fmoc-Ala was used, and as the last amino acid, Boc-Leu-OH·H₂O (249.3 mg, 1 mmol), from which a protecting group can be removed with an acid, was used. On the solid phase resin, a 23-residue peptide having a protecting group of Boc-Leu-Ala-Ala-Val-Ser(tBu)-Val-Asp(OtBu)-Cys(Trt)-Ser(t Bu)-Glu(OtBu)-Tyr(tBu)-Pro-Lys(Boc)-Pro-Ala-Cys(Trt)-Thr( tBu)-Met-Glu(OtBu)-Tyr(tBu)-Arg(Pbf)-Pro-Leu (SEQ ID NO:1) was obtained. To the resulting peptide, AcOH : DCM : MeOH = 5 : 4 : 1 (2 mL) was added, followed by stirring at room temperature for three hours. After stirring, the resin was removed by filtration and washed with MeOH. The filtrate was added to separately prepared hexane for crystallization. After filtration, the crystal thus obtained was subjected to azetropic with an excess amount of benzene three times, and then the resulting peptide was lyophilized (Figure 5, top. Note: the measurement was made after deprotection).

The peptide thus obtained (a 23-residue peptide having a protecting group as shown in SEQ ID NO:1) (39 mg, 10 µmol), MS4A, benzyl mercaptan (35.5 µL, 0.3 mmol) were stirred in a DMF solvent (1.35 mL) under a stream of argon at -20°C for one hour. Subsequently, PyBOP (26 mg, 50 µmol) and DIPEA (8.5 µL, 50 µmol) were added to the resulting mixture, followed by stirring for two hours. After stirring, an excess amount of diethyl ether was added to the reaction solution to precipitate a compound, followed by filtration. Thereafter, the precipitate thus obtained was dissolved in DMF. The resulting solution was concentrated under reduced pressure, to which a solution of 95% TFA, 2.5% TIPS, and 2.5% H₂O (1 mL) was added, followed by stirring at room temperature for two hours (Figure 5, middle). The resulting reaction solution was concentrated under reduced pressure and then purified by HPLC (Cadenza column CD18 (Imtakt Inc.), 3 mm, 75 x 4.6 mm, developing solvent A: a 0.09% aqueous solution of TFA B: 0.1% TFA acetonitrile : water = 90 : 10 gradient A : B = 80 : 20 → 40 : 60 (acetonitrile gradient: 18% → 54%) 15 minutes a flow rate of 1.0 mL/min) to give a 23-residue peptide having a benzyl thioester at its C-terminus which is Leu-Ala-Ala-Val-Ser-Val-Asp-Cys-Ser-Glu-Tyr-Pro-Lys-Pro-A la-Cys-Thr-Met-Glu-Tyr-Arg-Pro-Leu-SBn (SEQ ID NO:2) (Figure 5, bottom).
ESI-MS: Calcd for C₁₁₈H₁₈₁N₂₇O₃₄S₄: [M+2H]²⁺ 1326.0, Found. 1325.8

### [Synthesis of Fragment 2]

Subsequently, in a separate solid synthesis column, Amino-PEGA resin (the product of Merck) (1 g, 50 µmol) was placed, which was sufficiently washed with methylene chloride (DCM) and DMF. The resulting resin was sufficiently swelled in DMF. Then, 4-hydroxymethyl-3-methoxyphenoxybutyric acid (HMPB) (0.125 mmol), TBTU (0.125 mmol), and N-ethylmorpholine (0.125 mmol) were dissolved in DMF (1 ml) and the resulting mixture was poured into the column, followed by stirring at room temperature for two hours. The resin was sufficiently washed with DMF and DCM and the reaction was confirmed by Kaiser Test. The resin was confirmed to be negative (-) by Kaiser Test and swelled in DCM for one hour. HMPB-PEGA resin was obtained, which was used as a solid support for solid phase synthesis.

Fmoc-Phe (96.9 mg, 0.25 mmol), MSNT (74 mg, 0.25 mmol), and N-methylimidazole (14.9 µl, 0.188 mmol) were dissolved in DCM (1 mL) and the resulting mixture was poured in a solid phase synthesis column, followed by stirring at room temperature for two hours. After stirring, the resin was washed with DCM and DMF, and the Fmoc group was deprotected by treatment with a 20% piperidine/DMF solution (1 mL) for 20 minutes. The resulting product was washed with DMF and the reaction was confirmed with Kaiser Test. Thereafter, the peptide chain extension was carried out by sequentially condensing amino acids using the method shown below.

An amino acid having an amino group protected with a Fmoc group and HOBt (33.8 mg, 0.25 mmol), and DIPCI (38.5 µL, 0.25 mmol) were dissolved in DMF (1 mL) and the resulting solution was activated for 15 minutes. Thereafter, the solution was poured into the solid phase synthesis column, followed by stirring at room temperature for one hour. After stirring, the resin was washed with DCM and DMF. The Fmoc-group was deprotected with a 20% piperidine/DMF solution (1 mL) for 20 minutes. The above operation was repeated to sequentially condense amino acids. As the amino acid having a protected amino group, Fmoc-Asn, Fmoc-Cys(Trt), Fmoc-Lys(Boc), Fmoc-Asn, Fmoc-Gly, Fmoc-Tyr(tBu), Fmoc-Thr(tBu), and Fmoc-Lys(Boc) were used, and a 9-residue peptide having a protecting group which is
Fmoc-Lys(Boc)-Thr(tBu)-Tyr(tBu)-Gly-Asn-Lys(Boc)-Cys(Trt) -Asn-Phe (SEQ ID NO:3) was obtained on the solid phase resin. Then, 3 µmol equivalent of the 9-resiue peptide on the solid phase resin were transferred to another solid phase synthesis column and the Fmoc group was deprotected with a 20% piperidine/DMF solution (1 mL) for 20 minutes. The resin was sufficiently washed with DMF and transferred to an Eppendorf tube. Subsequently, an asparagine-linked sugar chain represented by the following formula (1) (12 mg, 6 µmol) and DEPBT (3 mg, 10 µmol) were dissolved in 0.20 mL of DMF : DMSO = 4 : 1, and the resulting mixture was transferred to the Eppendorf tube. To this tube, DIPEA (1.02 µL, 6 µmol) was added, followed by stirring at room temperature for 20 hours.

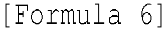

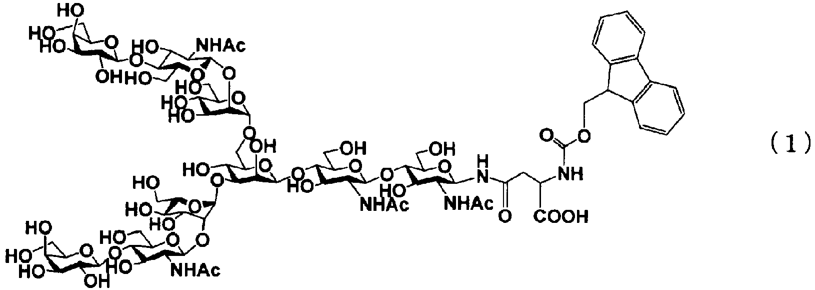

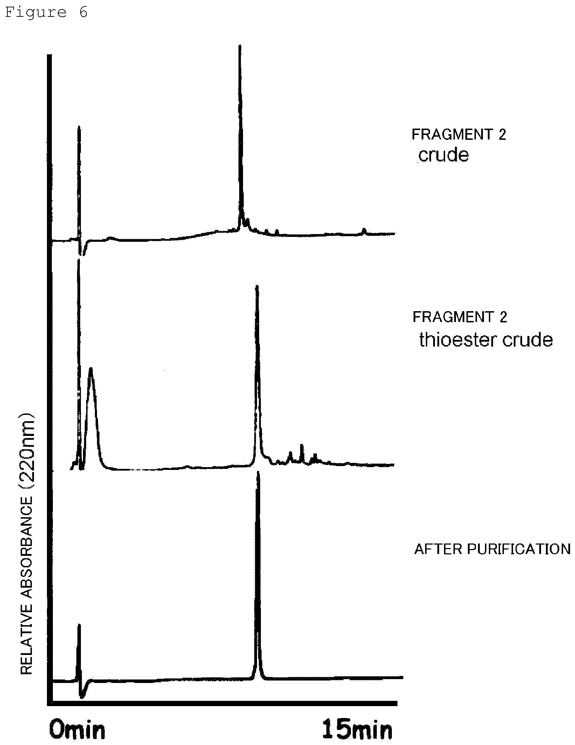

After stirring, the resin was transferred to a solid phase synthesis column and washed with DCM and DMF. The Fmoc-group was deprotected by treatment with a 20% piperidine/DMF solution (1 mL) for 20 minutes. The resulting product was washed with DMF. Thereafter, the glycopeptide chain extension was carried out by sequentially condensing amino acids using the method shown below. An amino acid having an amino group protected with a Fmoc group and HOBt (2 mg, 0.015 mmol), and DIPCI (2.3 µL, 0.015 mmol) were dissolved in DMF (0.375 mL) and the resulting solution was activated for 15 minutes. Thereafter, the solution was poured into the solid phase synthesis column, followed by stirring at room temperature for two hours. After stirring, the resin was washed with DCM and DMF. The Fmoc-group was deprotected with a 20% piperidine/DMF solution (1 mL) for 20 minutes. The above operation was repeated to sequentially condense amino acids. As the amino acid having a protected amino group, Fmoc-Asp(OtBu), Fmoc-Ser(tBu), Fmoc-Gly, and Boc-Cys (Thz) were used, and a 14-residue glycosylated peptide having a protecting group which is Boc-Cys(Thz)-Gly-Ser(tBu)-Asp(OtBu)-Asn(Oligosaccharide)-Lys(Boc)-Thr(tBu)-Tyr(tBu)-Gly-Asn-Lys(Boc)-Cys(Trt)-Asn-Phe (SEQ ID NO:4) was obtained on the solid phase resin. To the glycosylated peptide, 1 mL of acetic acid : trifluoroethanol (= 1 : 1) was added, followed by stirring at room temperature for 20 hours. The resin was removed by filtration and washed with MeOH, and the filtrate was concentrated under reduced pressure. The concentrated filtrate was subjected to azeotropic with benzene three times. The residue thus obtained was dissolved and then lyophilized (Figure 6, top. Note: the measurement was made after deprotection).

The peptide thus obtained (a 14-residue glycosylated peptide having a protecting group as shown in SEQ ID NO:4) (11.7 mg, 3 µmol), MS4A (10 mg), and benzyl mercaptan (10.6 µL, 0.09 mmol) were stirred in a DMF solvent (0.41 mL) under a stream of argon at -20°C for one hour. Subsequently, PyBOP (7.8 mg, 15 µmol) and DIPEA (2.6 µL, 15 µmol) were added to the resulting mixture, followed by stirring for two hours. After stirring, an excess amount of diethyl ether was added to the reaction solution to precipitate a compound, followed by filtration. Thereafter, the precipitate thus obtained was dissolved in DMF. The resulting solution was concentrated under reduced pressure, to which a solution of 95% TFA, 2.5% TIPS, and 2.5% H₂O (1 mL) was added, followed by stirring at room temperature for two hours (Figure 6, middle). The resulting reaction solution was concentrated under reduced pressure and then purified by HPLC (Cadenza column CD18 (Imtakt Inc.), 3 mm, 75 × 4.6 mm, developing solvent A: a 0.09% aqueous solution of TFA B: 0.1% TFA acetonitrile : water = 90 : 10 gradient A : B = 80 : 20 → 40 : 60 (acetonitrile gradient: 18% → 54%) 15 minutes, a flow rate of 1.0 mL/min) to give a 14-residue glycosylated peptide having a benzyl thioester at its C-terminus which is Cys(Thz)-Gly-Ser-Asp-Asn(Oligosaccharide)-Lys-Thr-Tyr-Gly -Asn-Lys-Cys-Asn-Phe-SBn (SEQ ID NO:5) (Figure 6, bottom).
ESI-MS: Calcd for C₁₃₃H₂₀₃N₂₃O₆₇S₃: [M+2H]²⁺ 1647.1, Found. 1646.6

### [Synthesis of Fragment 3]

Into a solid phase synthesis column, 2-chlorotrityl resin (200 µmol) was placed, which was then sufficiently washed with methylene chloride (DCM). Separately, DCM (1.2 mL) having Fmoc-Cys (Trt) (351.4 mg, 0. 6 mmol) and DIPEA (272.1 µL, 1.6 mmol) dissolved therein was prepared and poured into the solid phase synthesis column charged with the resin, followed by stirring at room temperature for two hours . After stirring, the resin was washed with DCM : MeOH : DIPEA = 17 : 2 : 1, DCM, and DMF. Subsequently, the Fmoc group was deprotected by treatment with a 20% piperidine/DMF solution (2 mL) for 20 minutes. The resulting product was washed with DMF and the reaction was confirmed with Kaiser Test. Thereafter, the peptide chain extension was carried out by sequentially condensing amino acids using the method shown below.

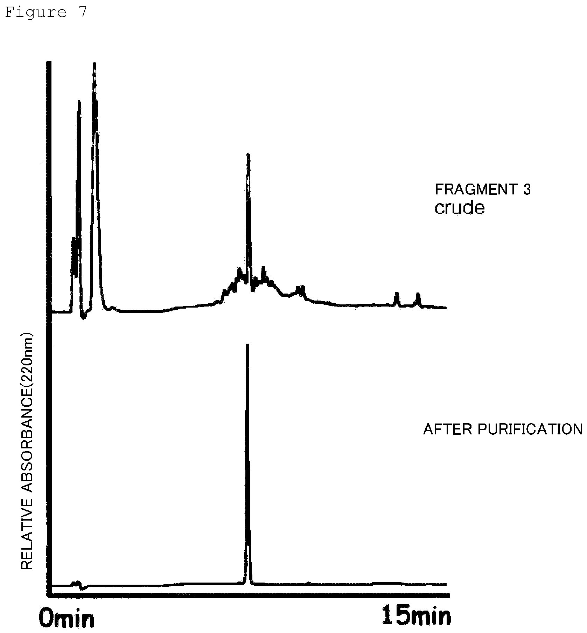

An amino acid having an amino group protected with a Fmoc group and HOBt (135.1 mg, 1 mmol) and DIPCI (153.9 µL, 1 mmol) were dissolved in DMF (4 mL) and the resulting solution was activated for 15 minutes. Thereafter, the solution was poured into the solid phase synthesis column, followed by stirring at room temperature for one hour. After stirring, the resin was washed with DCM and DMF. The Fmoc-group was deprotected by treatment with a 20% piperidine/DMF solution (2 mL) for 20 minutes. The above operation was repeated to sequentially condense amino acids. As the amino acid having a protected amino group, Fmoc-Lys(Boc), Fmoc-Gly, Fmoc-Phe, Fmoc-His(Trt), Fmoc-Ser(tBu), Fmoc-Leu, Fmoc-Thr(tBu), Fmoc-Leu, Fmoc-Thr(tBu), Fmoc-Gly, Fmoc-Asn, Fmoc-Ser(tBu), Fmoc-Glu(OtBu), Fmoc-Val, Fmoc-Val, Fmoc-Ala, Fmoc-Asn, and Fmoc-Cys(Trt) were used, and a 19-residue peptide having a protecting group which is Cys(Trt)-Asn-Ala-Val-Val-Glu(OtBu)-Ser(tBu)-Asn-Gly-Thr(t Bu) -Leu-Thr (tBu) -Leu-Ser (tBu) -His (Trt) -Phe-Gly-Lys (Boc) -C ys (Trt) (SEQ ID NO : 6) was obtained on the solid phase resin. To the peptide, a solution of 95% TFA, 2.5% TIPS, and 2.5% H₂O (3 mL) was added, followed by stirring at room temperature for two hours. Subsequently, the resin was removed by filtration and the filtrate was concentrated under reduced pressure (Figure 7, top). The concentrated filtrate was purified by HPLC (Cadenza column CD18 (Imtakt Inc.), 3 mm, 75 × 4.6 mm, developing solvent A: a 0.09% aqueous solution of TFA B: 0.1% TFA acetonitrile : water = 90 : 10 gradient A : B = 80 : 20 → 40 : 60 (acetonitrile gradient: 18% → 54%) 15 minutes a flow rate of 1.0 mL/min) to give a 19-residue peptide which is Cys-Asn-Ala-Val-Val-Glu-Ser-Asn-Gly-Thr-Leu-Thr-Leu-Ser-H is-Phe-Gly-Lys-Cys (SEQ ID NO:7) (Figure 7, bottom).
ESI-MS: Calcd for C₈₃H₁₃₄N₂₄O₂₈S₂: [M+2H]²⁺ 991.1, Found. 991.0

### [Ligation of Fragments 2 and 3 by NCL]

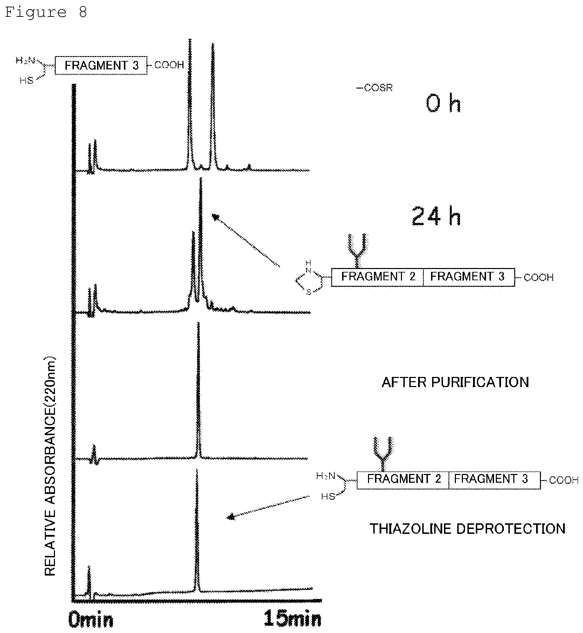

Two kinds of peptides, namely 1.9 mg (1 µmol) of Fragment 3 (a 19-residue peptide as shown in SEQ ID NO: 7) and 3.2 mg (1 µmol) of Fragment 2 (a 14-residue glycosylatedpeptide with a protecting group having a benzyl thioester at its C-terminus as shown in Figure 5) were placed in the same Eppendorf tube and dissolved in 485 µL of a 0.1% phosphate buffer (pH 7.5, containing 6M guanidine hydrochloride). Subsequently, thiophenol (15 µL) was added to the resulting mixture at 25°C, and reactions were allowed to proceed at room temperature (0h in Figure 8). After 24 hours, the completion of the reaction was confirmed by HPLC (24h in Figure 8). Subsequently, the reaction solution was purified by HPLC (Cadenza column CD18 (Imtakt Inc.), 3 mm, 75 × 4.6 mm, developing solvent A: a 0.09% aqueous solution of TFA B: 0.1% TFA acetonitrile : water = 90 : 10 gradient A : B = 80 : 20 → 40 : 60 (acetonitrile gradient: 18% → 54%) 15 minutes a flow rate of 1.0 mL/min) (Figure 8, After purification). Thereafter, the resulting peptide was lyophilized to give a 33-residue glycosylated peptide having a protecting group which is Cys(Thz)-Gly-Ser-Asp-Asn(Oligosaccharide)-Lys-Thr-Tyr-Gly -Asn-Lys-Cys-Asn-Phe-Cys-Asn-Ala-Val-Val-Glu-Ser-Asn-Gly-Thr-Leu-Thr-Leu-Ser-His-Phe-Gly-Lys-Cys (SEQ ID NO:8).
ESI-MS: Calcd for C₂₀₉H₃₂₉N₄₇O₉₅S₄: [M+4H]⁴⁺ 1287.28, Found. 1287.6

The peptide thus obtained (a 33-residue glycosylated peptide having a protecting group as shown in SEQ ID NO:8) was dissolved in a 0.2M aqueous solution of methoxyamine (pH = 4.0). After four hours, the completion of the reaction was confirmed by HPLC, and the resulting product was purified by HPLC (Cadenza column CD18 (Imtakt Inc.), 3 mm, 75 × 4.6 mm, developing solvent A: a 0.09% aqueous solution of TFA B: 0.1% TFA acetonitrile : water = 90 : 10 gradient A : B = 80 : 20 → 40 : 60 (acetonitrile gradient: 18% → 54%) 15 minutes a flow rate of 1.0 mL/min) (Figure 8, Thiazoline deprotection) . Thereafter, the resulting peptide was lyophilized to give a 33-residue glycosylated peptide which is Cys-Gly-Ser-Asp-Asn(Oligosaccharide)-Lys-Thr-Tyr-Gly-Asn-Lys-Cys-Asn-Phe-Cys-Asn-Ala-Val-Val-Glu-Ser-Asn-Gly-Thr-L eu-Thr-Leu-Ser-His-Phe-Gly-Lys-Cys (SEQ ID NO:9).
ESI-MS: Calcd for C₂₀₈H₃₂₉N₄₇O₉₅S₄: [M+4H]⁴⁺ 1284.28, Found. 1284.5

The 33-residue glycosylated peptide as shown in SEQ ID NO:9 was similarly obtained also under the following conditions.

Two kinds of peptides, namely 1.9 mg (1 µmol) of Fragment 3 (a 19-residue peptide as shown in SEQ ID NO: 7) and 3.2 mg (1 µmol) of Fragment 2 (a 14-residue glycosylated peptide having a protecting group and a benzyl thioester at its C-terminus as shown in Figure 5) were each placed in separate Eppendorf tubes and dissolved in 247.5 µL of a 0.1% phosphate buffer (pH 7.5, containing 6M guanidine hydrochloride). The contents were then combined together in one Eppendorf tube. Subsequently, 1% thiophenol (5 µL) was added to the resulting mixture at 25°C, and reactions were allowed to proceed at room temperature. The reaction was followed by HPLC and mass spectrometry, and disappearance of Fragment 3 was confirmed by HPLC after seven hours. Thereafter, a 0.2M aqueous solution of methoxyamine was added to bring the pH of the system to around 4 to deprotect the N-terminal Cys. The completion of the reaction was confirmed after six hours by mass spectrometry, and the resulting reaction solution was purified by HPLC (Cadenza column CD18 (Imtakt Inc.), 3 mm, 75 × 4.6 mm, developing solvent A: a 0.09% aqueous solution of TFA B: 0.1% TFA acetonitrile : water = 90 : 10 gradient A : B = 80 : 20 → 40 : 60 (acetonitrile gradient: 18% → 54%) 15 minutes a flow rate of 1.0 mL/min). The resulting peptide was lyophilized to give a 33-residue glycosylated peptide as shown in SEQ ID NO:9.
ESI-MS: Calcd for C₂₀₈H₃₂₉N₄₇O₉₅S₄: [M+4H]⁴⁺ 1284.28, Found. 1284.5

### [Ligation of Fragment 1 and Fragments 2 and 3 by NCL]

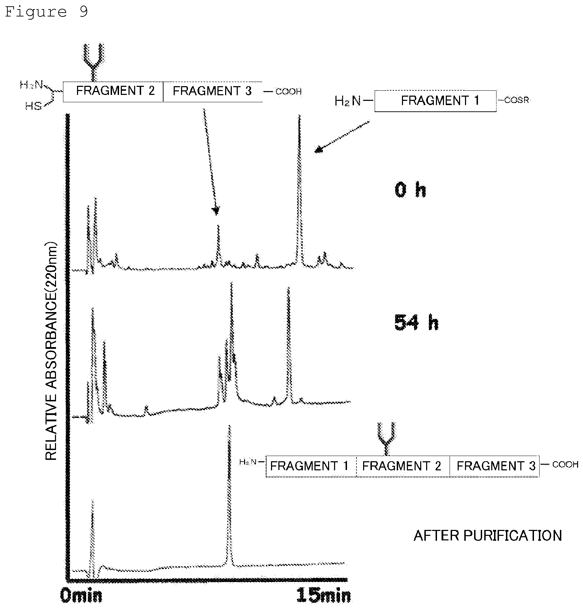

Two kinds of peptides, namely 1.3 mg (0.25 µmol) of the 33-residue glycosylated peptide prepared by ligating Fragments 2 and 3 and 1.3 mg (0.50 µmol) of Fragment 1 (a 23-residue peptide having a benzyl thioester at its C-terminus as shown in SEQ ID NO:2) were placed in the same Eppendorf tube and dissolved in 485 µL of a 0.1% phosphate buffer (pH 7.5, containing 8M guanidine hydrochloride). Subsequently, thiophenol (15 µL) was added to the resulting mixture at 25°C, and reactions were allowed to proceed at room temperature (0h in Figure 9). After 54 hours, the completion of the reaction was confirmed by HPLC (54h in Figure 9). Subsequently, the reaction solution was purified by HPLC (Cadenza column CD18 (Imtakt Inc.), 3 mm, 75 x 4.6 mm, developing solvent A: a 0.09% aqueous solution of TFA B: 0.1% TFA acetonitrile : water = 90 : 10 gradient A : B = 80 : 20 → 40 : 60 (acetonitrile gradient: 18% → 54%) 15 minutes a flow rate of 1.0 mL/min) (Figure 9, bottom). Thereafter, the resulting peptide was lyophilized to give a 56-residue glycosylated peptide of Leu-Ala-Ala-Val-Ser-Val-Asp-Cys-Ser-Glu-Tyr-Pro-Lys-Pro-A la-Cys-Thr-Met-Glu-Tyr-Arg-Pro-Leu-Cys-Gly-Ser-Asp-Asn(Ol igosaccharide)-Lys-Thr-Tyr-Gly-Asn-Lys-Cys-Asn-Phe-Cys-As n-Ala-Val-Val-Glu-Ser-Asn-Gly-Thr-Leu-Thr-Leu-Ser-His-Phe -Gly-Lys-Cys (SEQ ID NO:10).
ESI-MS: Calcd for C₃₁₉H₅₀₂N₇₄O₁₂₉S₇: [M+5H]⁵⁺ 1532.46, Found. 1532.7

The 56-residue glycosylated peptide (SEQ ID NO:10) was similarly obtained also under the following conditions.

Two kinds of peptides, namely 1.3 mg (0.25 µmol) of the 33-residue glycosylated peptide as shown in SEQ ID NO:9 and 1.3 mg (0.50 µmol) of Fragment 1 (the 23-residue peptide having a benzyl thioester at its C-terminus as shown in SEQ ID NO:2) were each placed in separate Eppendorf tubes and dissolved in 247.5 µL of a 0.1% phosphate buffer (pH 7.5, containing 8M guanidine hydrochloride). The contents were then combined together in one Eppendorf tube. The reaction was followed by HPLC and mass spectrometry, and after 30 hours, the resulting reaction solution was purified by HPLC (Cadenza column CD18 (Imtakt Inc.), 3 mm, 75 × 4.6 mm, developing solvent A: a 0.09% aqueous solution of TFA B: 0.1% TFA acetonitrile : water = 90 : 10 gradient A : B = 80 : 20 → 40 : 60 (acetonitrile gradient: 18% → 54%) 15 minutes a flow rate of 1.0 mL/min).

### [Glycoprotein folding]

Into an Eppendorf tube, 0.5 mg (65.2 nmol) of the 56-residue glycosylated peptide (SEQ ID NO:10) prepared as above was transferred, which was then dissolved in 100 µL of 0.6M tris buffer (pH = 8.7, containing 0.6 M guanidine hydrochloride and 6 mM EDTA). The resulting mixture was diluted with 500 µL of distilled water to fold the glycosylated third domain of ovomucoid.

### [Fractionation by HPLC]

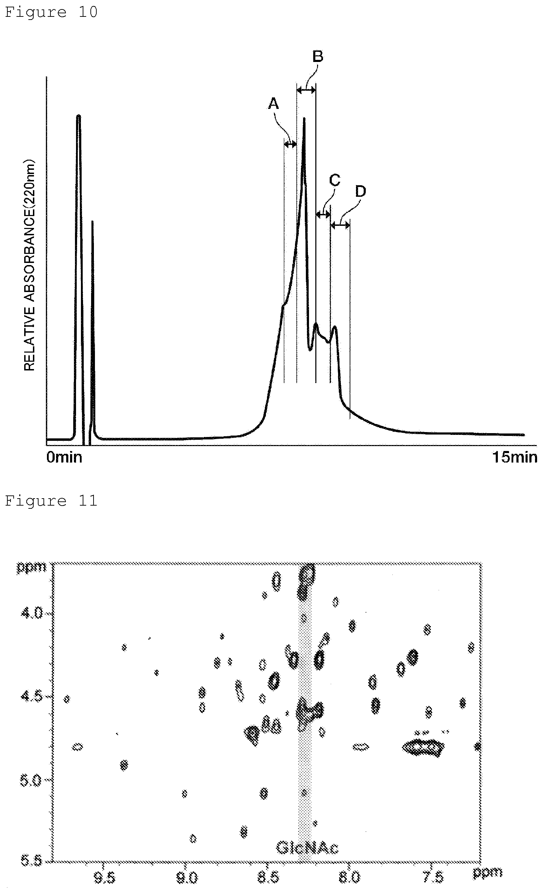

After 36 hours, the progress of the reaction was confirmed by HPLC and mass spectrometry, and the resulting product was purified by HPLC (Cadenza column CD18 (Imtakt Inc.), 3 mm, 75 × 4.6 mm, developing solvent A: a 0. 09% aqueous solution of TFA B: 0.1% TFA acetonitrile : water = 90 : 10 gradient A : B = 80 : 20 → 40 : 60 (acetonitrile gradient: 18% → 54%) 15 minutes a flow rate of 1.0 mL/min). As a result, four fractions A to D containing the 56-residue glycosylated peptide having a higher order structure of Leu-Ala-Ala-Val-Ser-Val-Asp-Cys-Ser-Glu-Tyr-Pro-Lys-Pro-A la-Cys-Thr-Met-Glu-Tyr-Arg-Pro-Leu-Cys-Gly-Ser-Asp-Asn(Ol igosaccharide)-Lys-Thr-Tyr-Gly-Asn-Lys-Cys-Asn-Phe-Cys-As n-Ala-Val-Val-Glu-Ser-Asn-Gly-Thr-Leu-Thr-Leu-Ser-His-Phe -Gly-Lys-Cys (SEQ ID NO:10) were obtained (Figure 10).
ESI-MS: Calcd for C₃₁₉H₅₀₂N₇₄O₁₂₉S₇: [M+5H ]⁵⁺ 1532.2, [M+4H]⁴⁺ 1915.0, [M+3H]³⁺ 2553.0,
A; Found. 1532.5, 1915.2, 2553.2
B; Found. 1532.5, 1915.2, 2553.2
C; Found. 1532.6, 1915.3, 2553.2
D; Found. 1532.7, 1915.4, 2553.3

The shift of the peak and the reduction of the mass from the bottom of Figure 9 to Figure 10 indicate formation of a disulfide bond through the aforementioned step of folding.

The reaction time can be appropriately changed (for example, 24 hours) by following the reaction by HPLC and mass spectrometry and confirming a change in the molecular weight and a change in the peak retention time by mass spectrometry and HPLC, respectively.

NMR measurement of Fraction B: A lyophilized Fraction B was dissolved in 5% D₂O/H₂O (300 µl) and 2D TOCSY was measured at 25°C, 60 ms, and 600 MHz. The resulting NMR spectrum is shown in Figure 11.

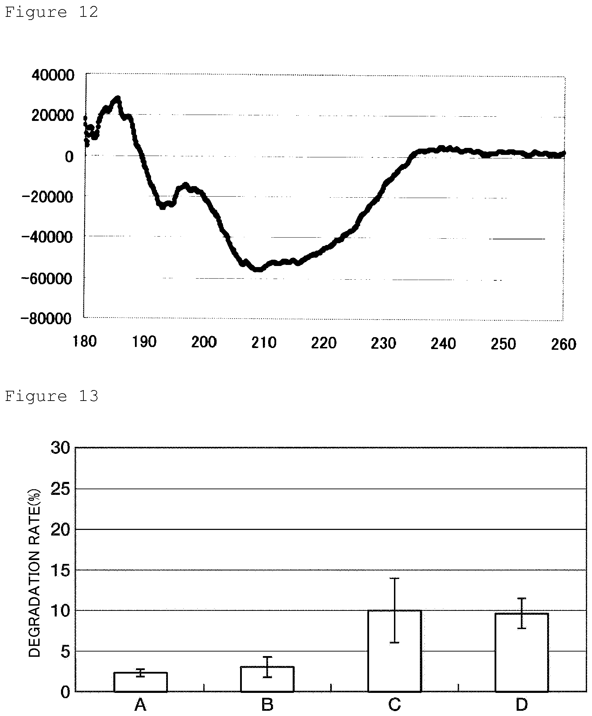

CD measurement of Fraction B: A lyophilized Fraction B was dissolved in distilled water and a CD measurement was performed. As an instrument, J-820 of JASCO Corporation was used. The measurement was performed within a range of 180 nm to 260 nm. The resulting CD spectrum is shown in Figure 12.

From Figure 11, it was confirmed that the glycopeptide having the same higher order structure could be highly purified only by separation by HPLC.

### 2. Chemical synthesis of the non-glycosylated third domain of silver pheasant ovomucoid

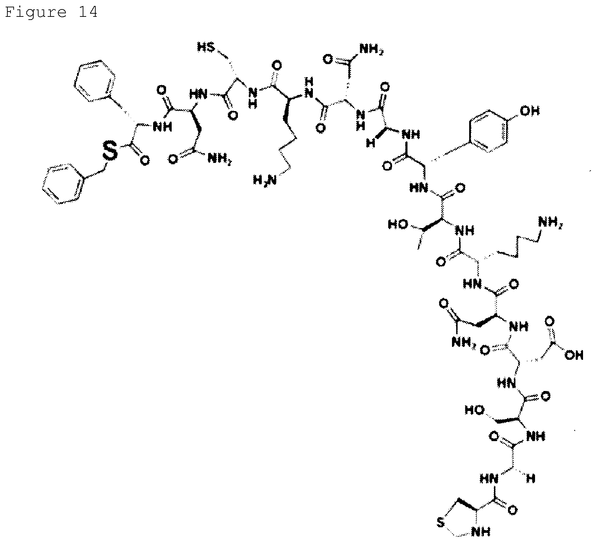

Similarly to Examples, three fragments were each synthesized and then ligated by NCL to synthesize a non-glycosylated third domain of silver pheasant ovomucoid. Fragments 1 and 3 were synthesized similarly to Examples. As shown in Figure 14, a fragment corresponding to Fragment 2 of Examples (hereinbelow referred to as "Fragment 2'") does not have a sugar chain as a Comparative Example.

### [Synthesis of Fragment 2']

Into a solid phase synthesis column, 2-chlorotrityl resin (143 mg, 200 µmol) was placed, which was then sufficiently washed with methylene chloride (DCM). Separately, DCM (1.2 mL) having Fmoc-Phe (232.4 mg, 0.6 mmol) and DIPEA (272.1 µL, 1.6 mmol) dissolved therein was prepared and poured into the solid phase synthesis column charged with the resin, followed by stirring at room temperature for two hours . After stirring, the resin was washed with DCM : MeOH : DIPEA = 17 : 2 : 1, DCM, and DMF. Subsequently, the Fmoc group was deprotected by treatment with a 20% piperidine/DMF solution (2 mL) for 20 minutes. The resulting product was washed with DMF and the reaction was confirmed with Kaiser Test. Thereafter, the peptide chain extension was carried out by sequentially condensing amino acids using the method shown below.

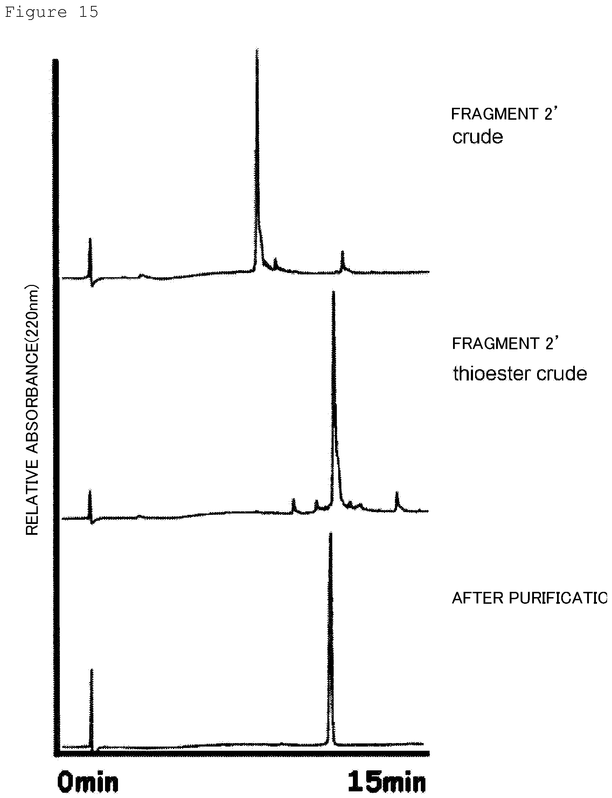

An amino acid having an amino group protected with a Fmoc group and HOBt (135.1 mg, 1 mmol) and DIPCI (153.9 µL, 1 mmol) were dissolved in DMF (0.4 mL) and the resulting solution was activated for 15 minutes. Thereafter, the solution was poured into the solid phase synthesis column, followed by stirring at room temperature for one hour. After stirring, the resin was washed with DCM and DMF. The Fmoc-group was deprotected by treatment with a 20% piperidine/DMF solution (1 mL) for 20 minutes. The above operation was repeated to sequentially condense amino acids. As the amino acid having a protected amino group, Fmoc-Asn, Fmoc-Cys(Trt), Fmoc-Lys(Boc), Fmoc-Asn, Fmoc-Gly, Fmoc-Tyr(tBu), Fmoc-Thr(tBu), Fmoc-Lys(Boc), Fmoc-Asn, Fmoc-Asp(OtBu), Fmoc-Ser (tBu), and Fmoc-Gly were used, and as the last amino acid, Boc-Cys(Thz)-OH (233.3 mg, 1 mmol), from which a protecting group can be removed with an acid, was used. On the solid phase resin, a 14-residue peptide having a protecting group of Boc-Cys (Thz) -Gly-Ser (tBu) -Asp (OtBu) -Asn-Lys (Boc) -Thr (tBu) -Tyr (tBu) -Gly-Asn-Lys (Boc) -Cys (Trt) -Asn-Phe (SEQ ID NO. 11) was obtained. To the resulting peptide, AcOH : DCM : MeOH = 5 : 4 : 1 (2 mL) was added, followed by stirring at room temperature for three hours. After stirring, the resin was removed by filtration and washed with MeOH. The filtrate was concentrated under reduced pressure. The concentrated filtratewas subjected to azetropic with an excess amount of benzene three times, and then the resulting peptide was lyophilized (Figure 15, top. Note: the measurement was made after deprotection).

In a DMF solvent (6.8 mL), 110 mg (50 µmol) of the peptide thus obtained (the 14-residue peptide having a protecting group as shown in SEQ ID NO: 11), MS4A (10 mg), benzyl mercaptan (177.4 µL, 1.5 mmol) were stirred under a stream of argon at -20°C for one hour. Subsequently, PyBOP (130 mg, 250 µmol) and DIPEA (42.5 µL, 250 µmol) were added to the resulting mixture, followed by stirring for two hours. After stirring, an excess amount of diethyl ether was added to the reaction solution to precipitate a compound, followed by filtration. Thereafter, the precipitate thus obtained was dissolved in DMF. The resulting solution was concentrated under reduced pressure, to which a solution of 95% TFA, 2.5% TIPS, and 2.5% H₂O (5 mL) was added, followed by stirring at room temperature for two hours (Figure 15, middle). The resulting reaction solution was concentrated under reduced pressure and then purified by HPLC (Cadenza column CD18 (Imtakt Inc.), 3 mm, 75 × 4.6 mm, developing solvent A: a 0.09% aqueous solution of TFA B: 0.1% TFA acetonitrile : water = 90 : 10 gradient A : B = 80 : 20 → 40 : 60 (acetonitrile gradient: 9% → 27%) 15 minutes a flow rate of 1.0 mL/min) to give 269.6 mg of a 14-residue peptide with a protecting group having a benzyl thioester at its C-terminus which is Cys(Thz)-Gly-Ser-Asp-Asn-Lys-Thr-Tyr-Gly-Asn-Lys-Cys-Asn-Phe-SBn (SEQ ID NO:12) (Figure 15, bottom).
ESI-MS: Calcd for C₇₁H₁₀₁N₁₉O₂₂S₃: [M+2H]²⁺ 834.8, Found. 834.7

### [Ligation of Fragment 2' and Fragment 3 by NCL]

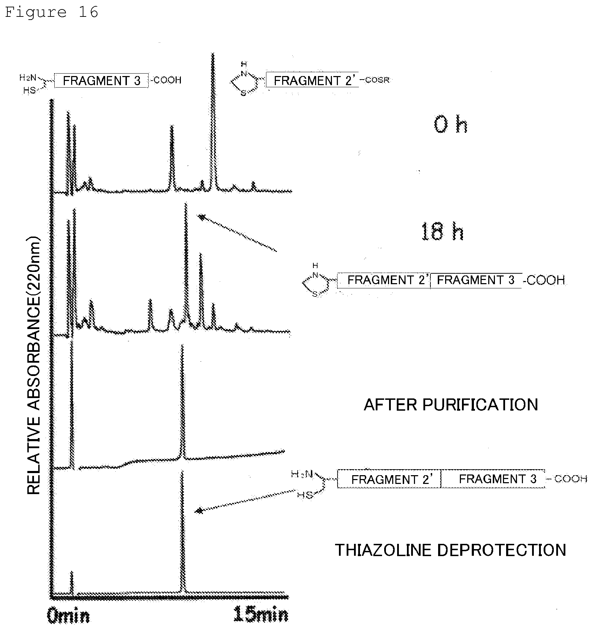

Two kinds of peptides, namely 1.6 mg (0.96 µmol) of Fragment 2' prepared as above (a 14-residue peptide with a protecting group having a benzyl thioester at its C-terminus as shown in SEQ ID NO: 12) and 1.9 mg (0.96 µmol) of Fragment 3 synthesized in Examples were placed in the same Eppendorf tube and dissolved in 495 µL of a 0.1% phosphate buffer (pH 7.5, containing 6M guanidine hydrochloride). Subsequently, thiophenol (5 µL) was added to the resulting mixture, and reactions were allowed to proceed at room temperature (0h in Figure 16). After 18 hours, the completion of the reaction was confirmed by HPLC (18h in Figure 16). Subsequently, the reaction solution was purified by HPLC (Cadenza column CD18 (Imtakt Inc.), 3 mm, 75 × 4.6 mm, developing solvent A: a 0.09% aqueous solution of TFA B: 0.1% TFA acetonitrile : water = 90 : 10 gradient A : B = 80 : 20 → 40 : 60 (acetonitrile gradient: 18% → 54%) 15 minutes a flow rate of 1.0 mL/min) (Figure 16, After purification). Thereafter, the resulting peptide was lyophilized to give a 33-residue peptide having a protecting group which is Cys(Thz)-Gly-Ser-Asp-Asn-Lys-Thr-Tyr-Gly-Asn-Lys-Cys-Asn-Phe-Cys-Asn-Ala-Val-Val-Glu-Ser-Asn-Gly-Thr-Leu-Thr-Leu-S er-His-Phe-Gly-Lys-Cys (SEQ ID NO:13).
ESI-MS: Calcd for C₁₄₇H₂₂₇N₄₃O₅₀S₄: [M+3H]³⁺ 1175.9, Found. 1175.4

The 33-residue peptide having a protecting group thus obtained was dissolved in a 0.2M aqueous solution of methoxyamine (pH = 4.0). After four hours, the completion of the reaction was confirmed by HPLC, and the resulting product was purified by HPLC (Cadenza column CD18 (Imtakt Inc.), 3 mm, 75 × 4.6 mm, developing solvent A: a 0. 09% aqueous solution of TFA B: 0.1% TFA acetonitrile : water = 90 : 10 gradient A : B = 80 : 20 → 40 : 60 (acetonitrile gradient: 18% → 54%) 15 minutes a flow rate of 1.0 mL/min) (Figure 16, Thiazoline deprotection). Thereafter, the resulting peptide was lyophilized to give a 33-residue peptide of Cys-Gly-Ser-Asp-Asn-Lys-Thr-Tyr-Gly-Asn-Lys-Cys-Asn-Phe-C ys-Asn-Ala-Val-Val-Glu-Ser-Asn-Gly-Thr-Leu-Thr-Leu-Ser-Hi s-Phe-Gly-Lys-Cys (SEQ ID NO:14).
ESI-MS: Calcd for C₁₄₆H₂₂₇N₄₃O₅₀S₄: [M+3H]³⁺ 1171.9, Found. 1171.5

The 33-residue peptide as shown in SEQ ID NO:14 was similarly obtained also under the following conditions.

Two kinds of peptides, namely 1.6 mg (0.96 µmol) of Fragment 2' (the 14-residue peptide having a benzyl thioester at its C-terminus as shown in SEQ ID NO: 12) and 1.9 mg (0.96 µmol) of Fragment 3 synthesized in Examples were each placed in separate Eppendorf tubes and dissolved in 247.5 µL of a 0.1% phosphate buffer (pH 7.5, containing 6M guanidine hydrochloride). The contents were then combined together in one Eppendorf tube. Subsequently, 1% thiophenol (5 µL) was added to the resulting mixture, and reactions were allowed to proceed at room temperature. The reaction was followed by HPLC and mass spectrometry, and disappearance of Fragment 3 was confirmed by HPLC after six hours. Thereafter, a 0.2M aqueous solution of methoxyamine was added to bring the pH of the system to around 4 to deprotect the N-terminal Cys. The completion of the reaction was confirmed after six hours by mass spectrometry, and the resulting reaction solution was purified by HPLC (Cadenza column CD18 (Imtakt Inc.), 3 mm, 75 × 4.6 mm, developing solvent A: a 0.09% aqueous solution of TFA B: 0.1% TFA acetonitrile : water = 90 : 10 gradient A : B = 80 : 20 → 40 : 60 (acetonitrile gradient: 18% → 54%) 15 minutes a flow rate of 1.0 mL/min).
ESI-MS: Calcd for C₁₄₆H₂₂₇N₄₃O₅₀S₄: [M+3H]³⁺ 1171.9, Found. 1171.5

### [Ligation of Fragment 1 and Fragments 2' and 3 by NCL]

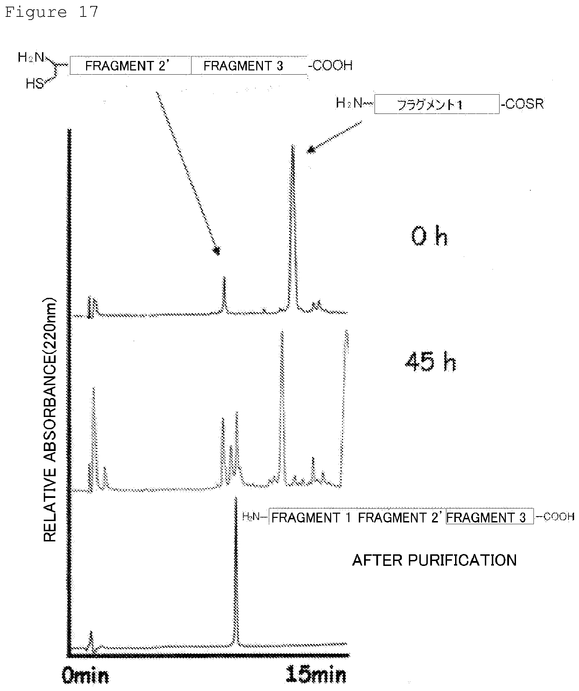

Two kinds of peptides, namely 0.6 mg (0.17 µmol) of the 33-residue peptide prepared as above and 1.1 mg (0.41 µmol) of Fragment 1 synthesized in Examples (the 23-residue peptide having a benzyl thioester at its C-terminus as shown in SEQ ID NO: 2) were placed in the same Eppendorf tube and dissolved in 485 µL of a 0.1% phosphate buffer (pH 7.5, containing 8M guanidine hydrochloride). Subsequently, thiophenol (15 µL) was added to the resulting mixture, and reactions were allowed to proceed at room temperature (0h in Figure 17). After 45 hours, the completion of the reaction was confirmed by HPLC (45h in Figure 17). Subsequently, the reaction solution was purified by HPLC (Cadenza column CD18 (Imtakt Inc.), 3 mm, 75 × 4.6 mm, developing solvent A: a 0.09% aqueous solution of TFA B: 0.1% TFA acetonitrile : water = 90 : 10 gradient A : B = 80 : 20 → 40 : 60 (acetonitrile gradient: 18% → 54%) 15 minutes a flow rate of 1.0 mL/min). Thereafter, the resulting peptide was lyophilized to give a 56-residue peptide of Leu-Ala-Ala-Val-Ser-Val-Asp-Cys-Ser-Glu-Tyr-Pro-Lys-Pro-A la-Cys-Thr-Met-Glu-Tyr-Arg-Pro-Leu-Cys-Gly-Ser-Asp-Asn-Ly s-Thr-Tyr-Gly-Asn-Lys-Cys-Asn-Phe-Cys-Asn-Ala-Val-Val-Glu -Ser-Asn-Gly-Thr-Leu-Thr-Leu-Ser-His-Phe-Gly-Lys-Cys (SEQ ID NO:15) (Figure 17, bottom).
ESI-MS: Calcd for C₂₅₇H₄₀₀N₇₀O₈₄S₇: [M+4H]⁴⁺ 1510.7, Found. 1510.6

That the 56-residue peptide as shown in SEQ ID NO: 15 was similarly obtained also under the following conditions.

Two kinds of peptides, namely 0.6 mg (0.17 µmol) of the 33-residue peptide as shown in SEQ ID NO: 14 and 1.1 mg (0.41 µmol) of Fragment 1 (the 23-residue peptide having a benzyl thioester at its C-terminus as shown in SEQ ID NO: 2) were each placed in separate Eppendorf tubes and dissolved in 247.5 µL of a 0.1% phosphate buffer (pH 7.5, containing 8M guanidine hydrochloride). The contents were then combined together in one Eppendorf tube. Subsequently, 1% thiophenol (5 µL) was added to the resulting mixture, and reactions were allowed to proceed at room temperature. The reaction was followed by HPLC and mass spectrometry, and after 30 hours, the resulting reaction solution was purified by HPLC (Cadenza column CD18 (Imtakt Inc.), 3 mm, 75 × 4.6 mm, developing solvent A: a 0.09% aqueous solution of TFA B: 0.1% TFA acetonitrile : water = 90 : 10 gradient A : B = 80 : 20 → 40 : 60 (acetonitrile gradient: 18% → 54%) 15 minutes a flow rate of 1.0 mL/min) . The resulting product was lyophilized to give the 56-residue peptide as shown in SEQ ID NO:15. ESI-MS: Calcd for C₂₅₇H₄₀₀N₇₀O₈₄S₇: [M+4H]⁴⁺ 1510.7, Found. 1510.6

### [Protein folding]

Into an Eppendorf tube, 0.4 mg (66.2 nmol) of the 56-residue peptide (SEQ ID NO:15) prepared as above was transferred, which was then dissolved in 100 µL of 0.6M tris buffer (pH = 8.7, containing 0.6 M guanidine hydrochloride and 6 mM EDTA). The resulting mixture was diluted with 500 µL of distilled water to fold the non-glycosylated third domain of ovomucoid.

### [Fractionation by HPLC]

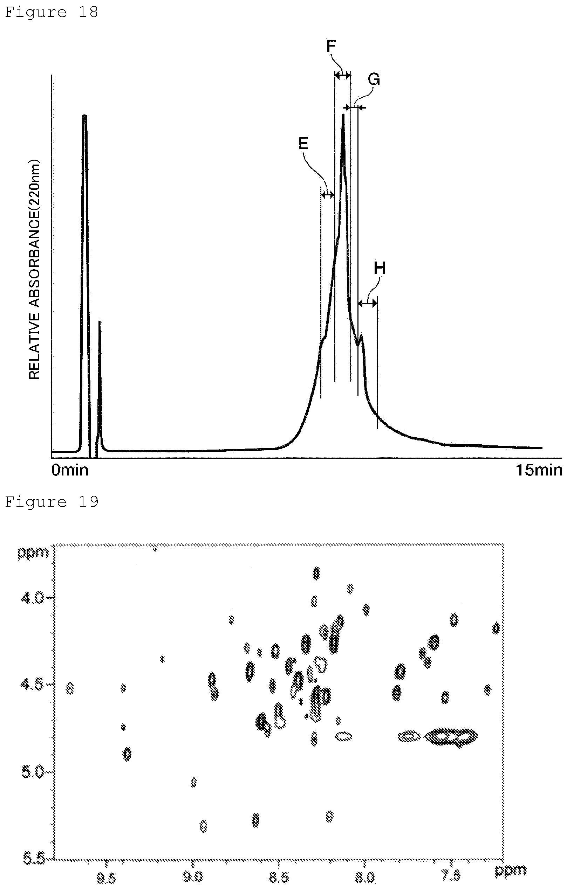

After 36 hours, the progress of the reaction was confirmed by HPLC and mass spectrometry, and the resulting product was purified by HPLC (Cadenza column CD18 (Imtakt Inc.), 3 mm, 75 × 4.6 mm, developing solvent A: a 0. 09% aqueous solution of TFA B: 0.1% TFA acetonitrile : water = 90 : 10 gradient A : B = 80 : 20 → 40 : 60 (acetonitrile gradient: 18% → 54%) 15 minutes a flow rate of 1.0 mL/min). As a result, four fractions E to H containing the 56-residue peptide having a higher order structure of Leu-Ala-Ala-Val-Ser-Val-Asp-Cys-Ser-Glu-Tyr-Pro-Lys-Pro-A la-Cys-Thr-Met-Glu-Tyr-Arg-Pro-Leu-Cys-Gly-Ser-Asp-Asn-Ly s-Thr-Tyr-Gly-Asn-Lys-Cys-Asn-Phe-Cys-Asn-Ala-Val-Val-Glu -Ser-Asn-Gly-Thr-Leu-Thr-Leu-Ser-His-Phe-Gly-Lys-Cys (SEQ ID NO:15) were obtained (Figure 18).
ESI-MS: Calcd for C₂₅₇H₃₉₄N₇₀O₈₄S₇: [M+5H ]⁵⁺ 1207.5, [M+4H ]⁴⁺ 1509.1, [M+3H]³⁺ 2011.9,
E; Found. 1207.7, 1509.3, 2012.0
F; Found. 1207.6, 1509.3, 2012.0
G; Found. 1207.7, 1509.3, 2012.0
H; Found. 1207.8, 1509.3, 2012.0

The shift of the peak and the reduction of the mass from the bottom of Figure 17 to Figure 18 indicate formation of a disulfide bond through the aforementioned step of folding.

The reaction time can be appropriately changed (for example, 24 hours) by following the reaction by HPLC and mass spectrometry and confirming a change in the molecular weight and a change in the peak retention time by mass spectrometry and HPLC, respectively.

NMR measurement of Fraction F: A lyophilized Fraction F was dissolved in 5% D₂O/H₂O (300 µl) and 2D TOCSY was measured at 25°C, 80 ms, and 600 MHz. The resulting NMR spectrum is shown in Figure 19.

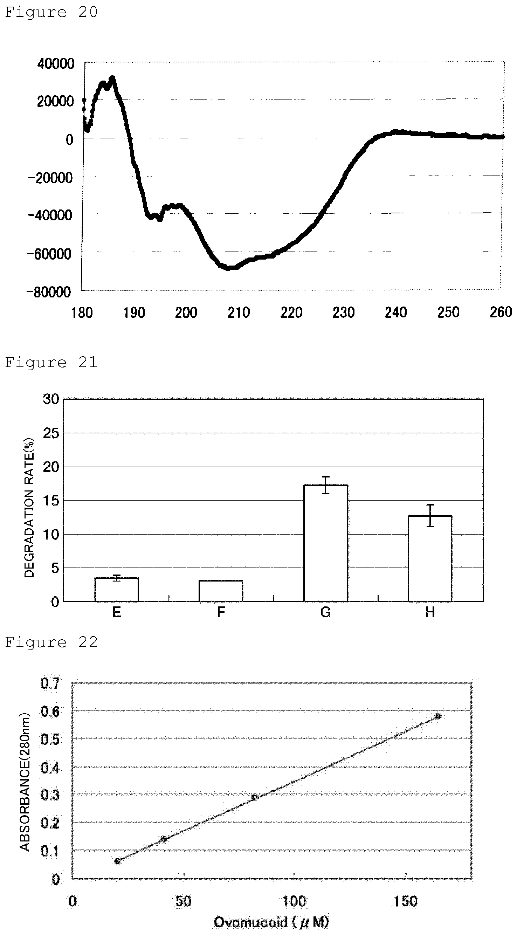

CD measurement of Fraction F: A lyophilized Fraction F was dissolved in distilled water and a CD measurement was performed. As an instrument, J-820 of JASCO Corporation was used. The measurement was performed within a range of 180 nm to 260 nm. The resulting CD spectrum is shown in Figure 20.

From Figure 19, it was confirmed that the peptide having same higher order structure could be highly purified only by separation by HPLC.

### [Production of a calibration curve for Fraction F]

Fraction F (1 mg) was dissolved in a 0.1 M phosphate buffer of pH 8.0 containing BSA (0.1 mg/ml). The resulting solution was diluted to prepare Fragment F having concentrations of 165 µM, 82.5 µM, 41.3 µM, and 20.6 µM. OD 280 of a solution of each concentration was measured three times. The values thus obtained were averaged out and shown in Table 1 and Figure 22.

**[Table 1]**

| ***µ*M** | **1time** | **2time** | **3time** | **average** |
|---|---|---|---|---|
| **165** | **0.58** | **0.57** | **0.58** | **0.57** |
| **82.5** | **0.27** | **0.29** | **0.3** | **0.28** |
| **41.2** | **0.14** | **0.13** | **0.14** | **0.14** |
| **20.6** | **0.06** | **0.06** | **0.06** | **0.06** |

### <Example 2> Measurement of the physiological activity [Measurement of the physiological activity of glycosylated OMSVP3 (Fractions A to D)]

An enzyme solution of a 0 .1 M phosphate buffer (pH =8.0, containing 0.01% α-chymotrypsin and 0.01% bovine serum albumin) and a substrate solution of a 0. 1 M phosphate buffer (pH= 8.0, containing 517 µM of a 14-residue peptide having a protecting group synthesized in Reference Example 1 (to be described later) (SEQ ID NO:16) and 0.01% bovine serum albumin) were prepared, and 20 µL of each solution was transferred to an Eppendorf tube. Separately, each of the Fractions A to D obtained in Example 1 was lyophilized and dissolved in 0.1 M phosphate buffer (pH = 8.0, containing 0.01% bovine serum albumin), and OD280 of each resulting solution was measured to prepare sample solutions of constant protein concentration. Then, 20 µL of each sample solution was added to the solution prepared as above and the inhibitory activity was measured. In this experiment, the final reaction concentration was as follows; a sample concentration of 2.5 µM, an enzyme concentration of 0.33 µg/mL, and a substrate concentration of 172 µM. The resulting reaction solutions were incubated at 37°C for 10 minutes, after which the reaction was terminated by addition of 5 µL of 4N hydrochloric acid. Similar operations were repeated three times, and an average degradation rate and a standard deviation were calculated for each sample. The results thus obtained are shown in Figure 13.

### [Measurement of the non-glycosylated physiological activity of OMSVP3 (Fractions E to H)]

An enzyme solution of a 0.1 M phosphate buffer (pH =8.0, containing 0.01% α-chymotrypsin and 0.01% bovine serum albumin) and a substrate solution of a 0. 1 M phosphate buffer (pH = 8.0, containing 517 µM of a 14-residue peptide having a protecting group synthesized in Reference Example 1 (to be described later) (SEQ ID NO:16) and 0.01% bovine serum albumin) were prepared, and 20 µL of each solution was transferred to an Eppendorf tube. Separately, each of the Fractions E to H obtained in Example 1 was lyophilized and dissolved in 0.1 M phosphate buffer (pH = 8.0, containing 0.01% bovine serum albumin), and OD280 of each resulting solution was measured to prepare sample solutions of constant protein concentration. Then, 20 µL of each sample solution was added to the solution prepared as above and the inhibitory activity was measured. In this experiment, the final reaction concentration was as follows; a sample concentration of 2.5 µM, an enzyme concentration of 0.33 µg/mL, and a substrate concentration of 172 µM. The resulting reaction solutions were incubated at 37°C for 10 minutes, after which the reaction was terminated by addition of 5 µL of 4N hydrochloric acid. Similar operations were repeated three times, and an average degradation rate and a standard deviation were calculated for each sample. The results thus obtained are shown in Figure 21.

### <Example 3> Measurement of the physiological activity

### (calculation of IC₅₀)

### [Calculation of IC₅₀ of glycosylated OMSVP3]

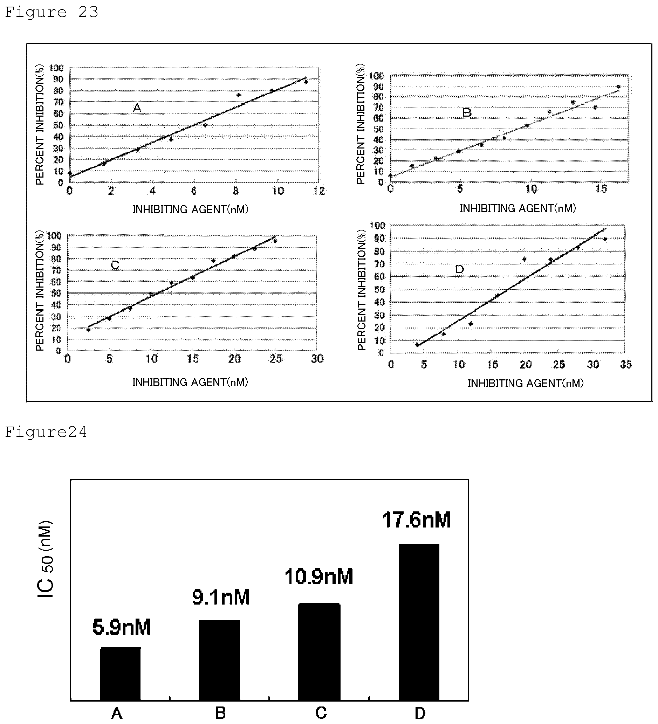

The 14-residue peptide having a protecting group synthesized in Reference Example 1 (to be described later) (SEQ ID NO:16) (1.5 mg) was dissolved in 1 mL of a 0.1 M phosphate buffer (pH 8.0, containing 0.1 mg/mL BSA) to prepare a 1 mM solution. The solution thus obtained was diluted to 0.34 mM using an absorption spectrometer (solution 1). Chymotrypsin (1 mg) was dissolved in 1 mL of a 0.1 M phosphate buffer (pH 8.0, containing 0.1 mg/mL BSA). The resulting solution was diluted 10-fold, and further diluted 10-fold. The above operation was repeated so that a solution of 0.2 µg/mL was prepared (solution 2). Fraction B was dissolved in 100 µL of a 0. 1 M phosphate buffer (pH 8.0, containing 0.1 mg/mL BSA), and the solution thus obtained was diluted to 65 nM using an absorption spectrometer. The resulting solution was diluted to prepare solutions of 58.5 nM, 52 nM, 45.5 nM, 39 nM, 32.5 nM, 26 nM, 19.5 nM, 13 nM, and 6.5 nM (solution 3). Into the same Eppendorf tube, 80 µ L of Solution 1 that was sufficiently cooled on ice and 40 µL each of Solutions 2 and 3 were transferred, followed by incubation for one hour at 37°C. After one hour, the reaction was terminated by addition of 16 µL of 1N hydrochloric acid. Then, 20 µL of the resulting reaction solution was mixed with 80 µL of buffer to make up a total of 100 µL, which was then measured by HPLC. A degradation rate per unit time (a reaction rate per unit time) was calculated from the peak area of HPLC of the reaction product. Figure 23 shows graphs plotting the percent inhibition with respect to each concentration of the inhibiting agent. Similarly, with regard also to Fractions A, C, and D, graphs were plotted in such a way that the concentrations of the inhibiting agent sandwiched the concentration at which the enzyme activity was inhibited by 50%. The resulting graphs are shown (Figure 23). The IC₅₀ values of glycosylated OMSVP3 (Fractions A to D) calculated based on these graphs are shown in a graph (Figure 24).

### [Calculation of IC₅₀ of non-glycosylated OMSVP3]

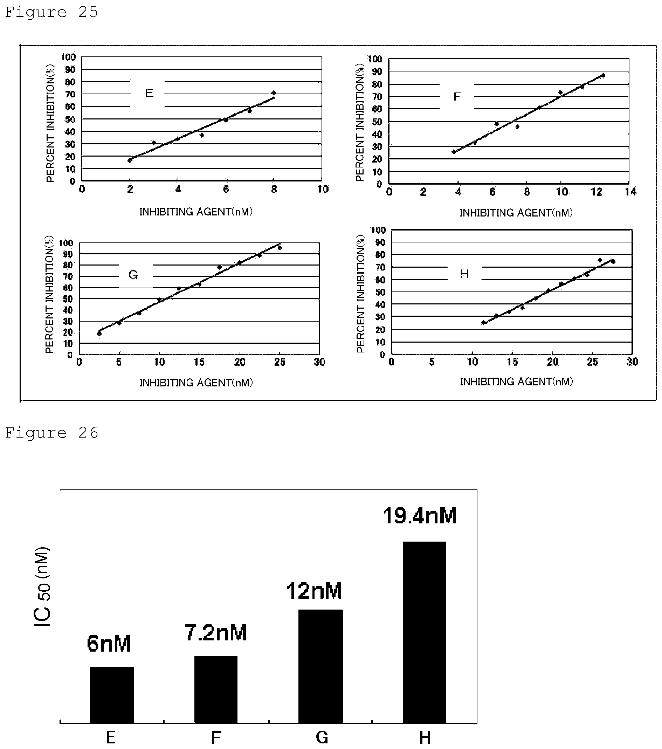

The 14-residue peptide having a protecting group synthesized in Reference Example 1 (to be described later) (SEQ ID NO:16) (1.5 mg) was dissolved in 1 mL of a 0.1 M phosphate buffer (pH8.0, containing 0.1 mg/mL BSA) to prepare a 1 mM solution. The solution thus obtained was diluted to 0.34 mM using an absorption spectrometer (solution 1). Chymotrypsin (1 mg) was dissolved in 1 mL of a 0.1 M phosphate buffer (pH 8.0, containing 0.1 mg/mL BSA). The resulting solution was diluted 10-fold, and further diluted 10-fold. The above operation was repeated so that a solution of 0.2 µg/mL was prepared (solution 2). Fraction F was dissolved in 100 µL of a 0.1 M phosphate buffer (pH 8.0, containing 0.1 mg/mL BSA), and the solution thus obtained was diluted to 65 nM using an absorption spectrometer. The resulting solution was diluted to prepare solutions of 58.5 nM, 52 nM, 45.5 nM, 39 nM, 32.5 nM, 26 nM, 19.5 nM, 13 nM, and 6.5 nM (solution 3). Into the same Eppendorf tube, 80 µ L of Solution 1 that was sufficiently cooled on ice and 40 µL each of Solutions 2 and 3 were transferred, followed by incubation for one hour at 37°C. After one hour, the reaction was terminated by addition of 16 µL of 1N hydrochloric acid. Then, 20 µL of the resulting reaction solution was mixed with 80 µL of buffer to make up a total of 100 µL, which was then measured by HPLC. A degradation rate per unit time (a reaction rate per unit time) was calculated from the peak area of HPLC of the reaction product. Figure 25 shows graphs plotting the percent inhibition with respect to each concentration of the inhibiting agent. Similarly, with regard also to Fractions E, G, and H, graphs were plotted in such a way that the concentrations of the inhibiting agent sandwiched the concentration at which the enzyme activity was inhibited by 50%. The resulting graphs are shown (Figure 25). The IC₅₀ values of non-glycosylated OMSVP3 (Fractions E to F) calculated based on these graphs are shown in a graph (Figure 26).

### <Example 4> Measurement of heat stability

A cell for CD measurement was filled with distilled water and then measured at room temperature. The measurement value thus obtained was provided as a blank, and all the measurement values obtained thereafter were calculated by subtracting the value of blank.

### [Heat stability of glycosylated OMSVP3 (Fraction B)]

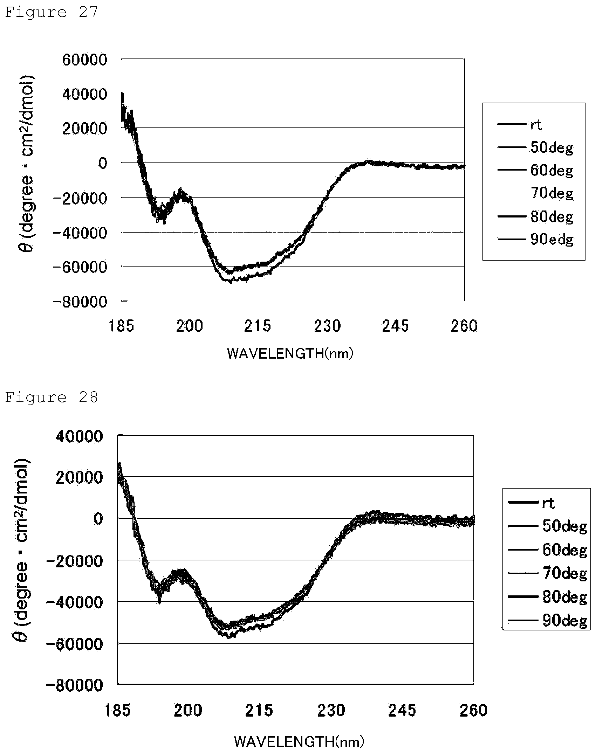

Fraction B was dissolved in 300 µL of distilled water and then measured at room temperature. After the measurement, the cell containing the sample was immersed in a constant temperature bath to carry out a variable temperature experiment. Firstly, the cell was immersed in a constant temperature bath at 50°C for 10 minutes and then left to stand at room temperature for 10 minutes, and then a measurement was made. Thereafter, in a similar operation, the CD spectrum was measured up to 90°C. The results thus obtained are shown in Figure 27.

### [Heat stability of non-glycosylated OMSVP3 (Fraction F)]

Fraction F was dissolved in 300 µL of distilled water and then measured at room temperature. After the measurement, the cell containing the sample was immersed in a constant temperature bath to carry out a variable temperature experiment. Firstly, the cell was immersed in a constant temperature bath at 50°C for 10 minutes and then left to stand at room temperature for 10 minutes, and then a measurement was made. Thereafter, in a similar operation, the CD spectrum was measured up to 90°C. The results thus obtained are shown in Figure 28.

### <Example 5> Disulfide mapping of the ovomucoid third domain

Synthesized OMSVP3 contains three disulfide bonds. A disulfide bond is formed during protein folding, and the formation process of a disulfide bond is an equilibrium reaction. Thus, a disulfide bond is considered to be possibly formed at a position different from a naturally-occurring protein.

It was predicted from the results of NMR and the evaluation of the inhibitory activity that the OMSVP3 (Fraction 3) synthesized herein was a single compound, and thus a disulfide bond was formed at the same position as a naturally-occurring protein. In view of the above, the following study was conducted to confirm whether a disulfide bond was surely formed in Fraction B at the same position as a naturally-occurring protein.

### [Disulfide mapping of OMSVP3 having uniform amino acid sequence and sugar chain (Fraction B)]

Cyanogen bromide (1 mg/mL) was reacted with Fraction B obtained in Example 1 (0.4 mg) in an aqueous solution of 40% acetonitrile and 2% TFA at 37°C overnight in a light-shielded condition. The resulting product was lyophilized and purified by HPLC (VyDAC column C4 (Imtakt Inc.), 3 µm, 4.5 × 250 mm, developing solvent A: a 0.09% aqueous solution of TFA B: 0.1% TFA acetonitrile : water = 90 : 10 gradient A : B = 80 : 20 → 40 : 60 (acetonitrile gradient: 18% → 54%) 30 minutes a flow rate of 1.0 mL/min) to give Fraction I. ESI-MS: Calcd for C₃₁₈H₄₉₄N₇₄O₁₃₀S₆: [M+4H]⁴⁺, 1907.5, Found. 1907.4

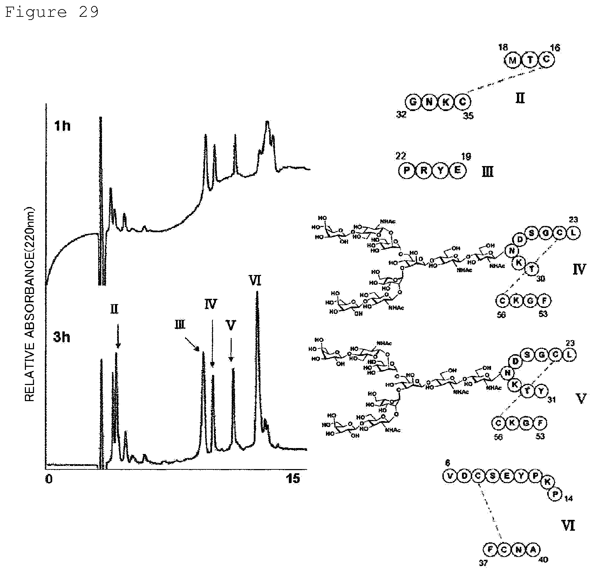

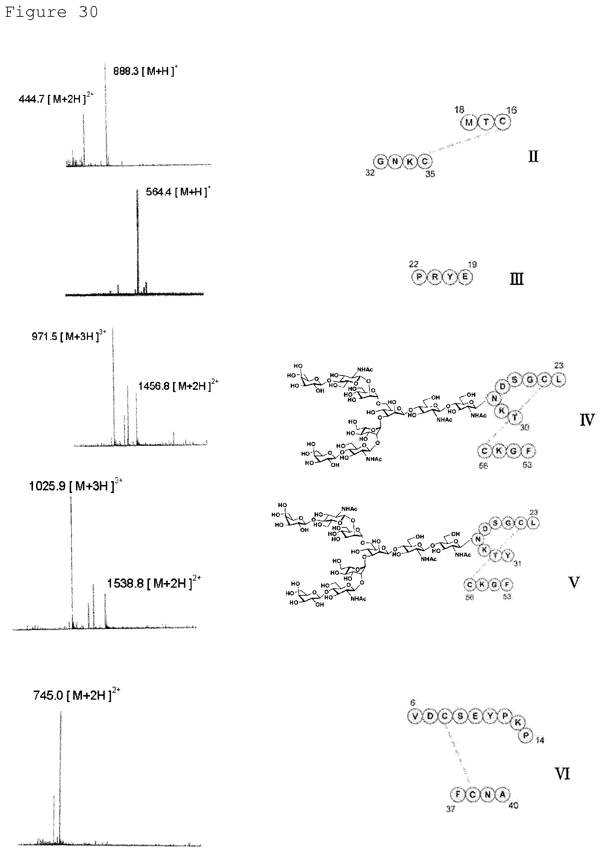

Then, Fraction I (0.1 mg) was dissolved in a 50 mM tris buffer (pH 7.6, containing 10 mM CaCl₂) having thermolysin (50 µg/mL) dissolved therein, and then incubated at 37°C. After three hours, the resulting mixture was purified by HPLC (VyDAC column C4 (Imtakt Inc.), 3 µm, 4.5 × 250 mm, developing solvent A: a 0.09% aqueous solution of TFA B: 0.1% TFA acetonitrile : water = 90 : 10 gradient A : B = 95 : 5 → 50 : 50 (acetonitrile gradient: 4.5% → 45%) 15 minutes a flow rate of 1.0 ml/min) to give Fractions II to VI (Figures 29 and 30).
Fraction II; Calcd for C₃₅H₅₄N₁₀O₁₃S₂: [M+2H]²⁺, 444.8, Found 444.7
Fraction III; Calcd for C₂₅H₃₇N₇O₈: [M+H]⁺, 564.4, Found 564.4 Fraction IV; Calcd for C₁₁₄H₁₈₇N₁₉O₆₄S₂: [M+3H]³⁺, 971.6, Found 971.5
Fraction V; Calcd for C₁₂₃H₁₉₆N₂₀O₆₆S₂: [M+3H]³⁺, 1026.0, Found 1025.9
Fraction VI; Calcd for C₆₄H₉₃N₁₅O₂₂S₂: [M+2H]²⁺, 744.8, Found 745.0

### [Disulfide mapping of non-glycosylated OMSVP3 (Fraction F)]

Cyanogen bromide (1 mg/mL) was reacted with Fraction F obtained in Example 1 (0.4 mg) in an aqueous solution of 40% acetonitrile and 2% TFA at 37°C overnight in a light-shielded condition. The resulting product was lyophilized and purified by HPLC (VyDAC column C4 (Imtakt Inc.), 3 µm, 4.5 × 250 mm, developing solvent A: a 0.09% aqueous solution of TFA B: 0.1% TFA acetonitrile : water = 90 : 10 gradient A : B = 80 : 20 → 40 : 60 (acetonitrile gradient: 18% → 54%) 30 minutes a flow rate of 1.0 mL/min) to give Fraction VII. ESI-MS: Calcd for C₂₅₆H₃₉₂N₇₀O₈₅S₆: [M+4H]⁴⁺, 1501.6, Found. 1501.5

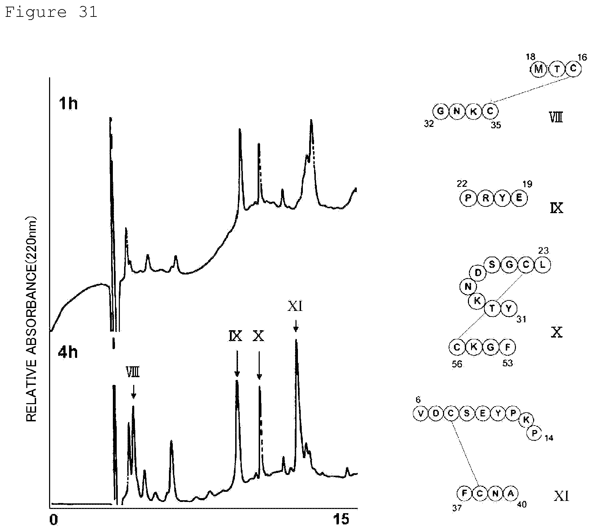

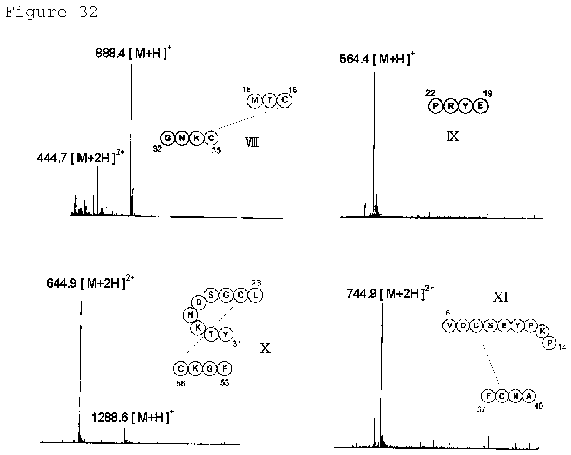

Then, Fraction VII (0.1 mg) was dissolved in a 50 mM tris buffer (pH 7.6, containing 10 mM CaCl₂) having thermolysin (50 µg/mL) dissolved therein, and then incubated at 37°C. After four hours, the resulting mixture was purified by HPLC (VyDAC column C4 (Imtakt Inc.), 3 µm, 4.5 × 250 mm, developing solvent A: a 0.09% aqueous solution of TFA B: 0.1% TFA acetonitrile : water = 90 : 10 gradient A : B = 95 : 5 → 50 : 50 (acetonitrile gradient: 4.5% → 45%) 15 minutes a flow rate of 1.0 ml/min) to give Fractions VIII to XI (Figures 31 and 32).
ESI-MS:
Fraction VIII; Calcd for C₃₅H₅₄N₁₀O₁₃S₂: [M+2H]²⁺, 444.8, Found 444.7
Fraction IX; Calcd for C₂₅H₃₇N₇O₈: [M+H]⁺, 564.4, Found 564.4
Fraction X; Calcd for C₅₂H₈₅N₁₅O₁₉S₂: [M+2H]²⁺, 644.8, Found 644.9
Fraction XI; Calcd for C₆₄H₉₃N₁₅O₂₂S₂: [M+2H]²⁺, 744.8, Found 744.9

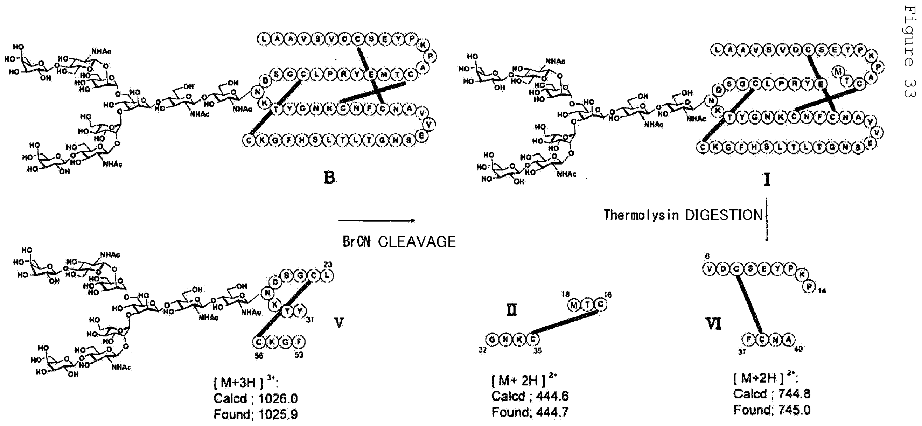

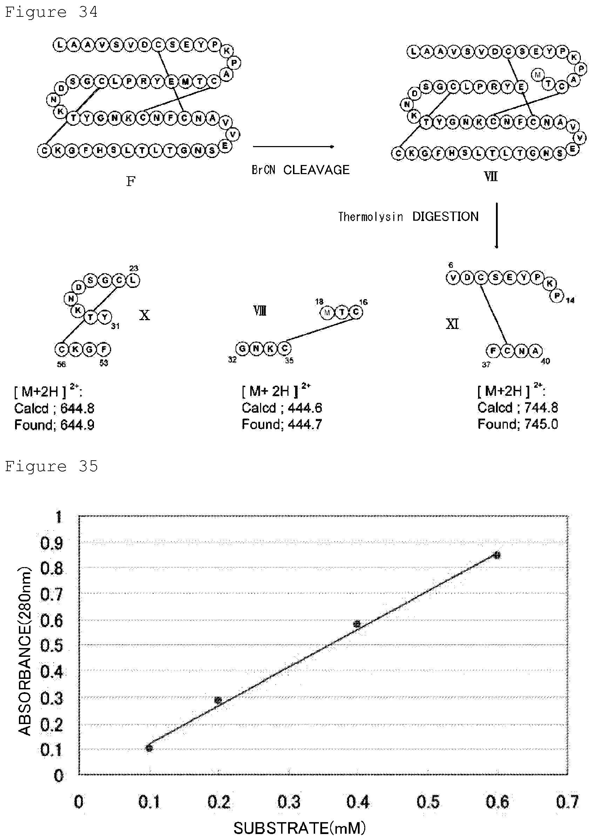

The peptide chain was specifically cleaved at a methionine position in the sequence of glycosylated OMSVP3 (Fraction B) by treatment with CNBr (Fraction I), and subsequently digested with thermolysin. As a result, peptide fragments linked by a disulfide bond were obtained (Figure 29). Each peptide fragment was purified and measured for mass by ESI-mass. Subsequently, an analysis was conducted to find out to which fragment of OMSVP3 the mass thus obtained corresponded. The proposed structure thereby obtained is shown in the bottom of Figure 33. Using the similar method, the non-glycosylated OMSVP3 (Fragment F) was analyzed in a similar manner (refer to Figures 31 and 34), and the proposed structure thereby obtained is shown in the bottom of Figure 34. It was confirmed that the disulfide bond was formed at the same position as naturally-occurring OMSVP3 (Figures 31 and 34). The position of disulfide bond in Fractions B and F proposed by the disulfide mapping corresponded to the position of disulfide bond in naturally-occurring OMSVP3, which had been already analyzed.

Based on the above findings, it was suggested that a glycoprotein having uniform amino acid sequence, sugar chain structure, and higher order structure could be produced by the steps of folding, fractionating, and collecting of the present invention. Also, in the case of OMSVP3, the fraction obtained as the maximum peak in the step of fractioning of the present invention had the same disulfide bond as naturally-occurring OMSVP3. Also, the faction was highly active, and a glycoprotein having a desired structure and activity could be efficiently produced by this fraction. The aforementioned finding also indicates that, with regard to the case in which another glycoprotein is produced, even when the maximum peak fraction has neither desired activity nor desired structure, a glycoprotein having uniform amino acid sequence, sugar chain structure, and higher order structure and having a predetermined activity can still be produced by appropriately collecting other factions having a desired activity, and this does not prevent the practicability of the present invention in any way.

In Examples of the present invention, the pattern obtained by fractionating the folded third domain of ovomucoid having a glycoprotein by HPLC (Figure 10) and the pattern obtained by fractionating the folded non-glycosylated third domain by HPLC (Figure 18) both had four weeks and were relatively similar. Further, considering also that both exhibited similar activity intensity, that is, the intensity was each found to be Fraction A > Fraction B > Fraction D > Fraction C, and Fraction F > Fraction E > Fraction H > Fraction G (Figures 13 and 21) in Example 2, and Fraction A > Fraction B > Fraction C > Fraction D, and Fraction E > Fraction F > Fraction G > Fraction H (Figures 23 to 26) in Example 3, it seemed that a protein having uniform higher order structure was eluted in the same order. The finding such that the position of disulfide bond in Fractions B and F, both of which had a high activity and were obtained as the maximum peak among other fractions, was the same was also consistent with the aforementioned findings.

However, the CD spectra of Fractions B and F did not match, suggesting that these fractions have different higher order structures (Figures 12 and 20). This suggests that addition of a sugar chain may possibly change the higher order structure of a protein by causing distortion in the folding of a non-glycosylated protein, and the like.

It is predicted that such an alteration in the higher order structure of a protein could affect the physiological activity considering that it also affects the binding ability of the protein to a substrate and the like, and further, it could also affect the blood half-life considering that it also affects the permeability of the glomerular filtration, and the like. Based on the above, it is understood that, when using a glycoprotein as a pharmaceutical product, it is important to only purify and separate a protein having a constant higher order structure in the state in which it is glycosylated to produce a medicine exerting a constant physiological activity and blood half-life. In this regard, the present invention enables this.

### <Reference Example 1 Synthesis of a substrate for the inhibitory activity test>

### [Synthesis of a substrate peptide]

Into a solid phase synthesis column, 2-chlorotrityl resin (143 mg, 200 µmol) was placed, which was then sufficiently washed with methylene chloride (DCM). Separately, DCM (1.2 mL) having Fmoc-Phe (232.4 mg, 0.6 mmol) and DIPEA (272.1 µL, 1.6 mmol) dissolved therein was prepared and poured into the solid phase synthesis column charged with the resin, followed by stirring at room temperature for two hours . After stirring, the resin was washed with DCM : MeOH : DIPEA = 17 : 2 : 1, DCM, and DMF. Subsequently, the Fmoc group was deprotected by treatment with a 20% piperidine/DMF solution (2 mL) for 20 minutes. The resulting product was washed with DMF and the reaction was confirmed with Kaiser Test. Thereafter, the peptide chain extension was carried out by sequentially condensing amino acids using the method shown below.

An amino acid having an amino group protected with a Fmoc group and HOBt (135.1 mg, 1 mmol) and DIPCI (153.9 µL, 1 mmol) were dissolved in DMF (0.4 mL) and the resulting solution was activated for 15 minutes. Thereafter, the solution was poured into the solid phase synthesis column, followed by stirring at room temperature for one hour. After stirring, the resin was washed with DCM and DMF. The Fmoc-group was deprotected by treatment with a 20% piperidine/DMF solution (1 mL) for 20 minutes. The above operation was repeated to sequentially condense amino acids. As the amino acid having a protected amino group, Fmoc-Asn, Fmoc-Cys(Trt), Fmoc-Lys(Boc), Fmoc-Asn, Fmoc-Gly, Fmoc-Tyr(tBu), Fmoc-Thr(tBu), Fmoc-Lys(Boc), Fmoc-Asn, Fmoc-Asp(OtBu), Fmoc-Ser (tBu), and Fmoc-Gly were used, and as the last amino acid, Boc-Cys(Thz)-OH (233.3 mg, 1 mmol), was used. On the solid phase resin, a 14-residue peptide having a protecting group of Boc-Cys (Thz) -Gly-Ser (tBu) -Asp (OtBu) -Asn-Lys (Boc) -Thr (tBu) -Tyr (tBu) -Gly-Asn-Lys (Boc) -Cys (Trt) -Asn-Phe (SEQ ID NO. 11) was obtained. To the resulting peptide, a solution containing 95% TFA, 2.5% TIPS, and 2.5% H₂O (3 mL) was added, followed by stirring at room temperature for two hours . After stirring, the resin was removed by filtration and the filtrate was concentrated under reduced pressure. The concentrated filtrate was purified by HPLC (VyDAC column C4 (Imtakt Inc.), 3 µm, 4.5 x 250 mm, developing solvent A: a 0.09% aqueous solution of TFA B: 0.1% TFA acetonitrile : water = 90 : 10 gradient A : B = 95 : 5 20 → 50 : 50 (acetonitrile gradient: 4.5% → 45%) 15 minutes a flow rate of 1.0 mL/min). The resulting product was lyophilized to give a 14-residue peptide having a protecting group of Cys(Thz)-Gly-Ser-Asp-Asn-Lys-Thr-Tyr-Gly-Asn-Lys-Cys-Asn-Phe (SEQ ID NO:16).
ESI-MS: Calcd for C₆₄H₉₅N₁₉O₂₃S₂: [M+2H]²⁺, 782.34, Found. 782.2

### [Production of a calibration curve for a substrate peptide]

Into 1 mL of a 0.1 M phosphate buffer (pH 8.0, containing BSA (0.1 mg/ml)), 1.5 mg of the 14-residue peptide thus synthesized was dissolved. The resulting mixture was diluted to prepare solutions having substrate concentrations of 0.6 mM, 0.4 mM, 0.2 mM, and 0.1 mM. OD 280 of a solution of each concentration was measured. The values thus obtained were averaged out and shown in Table 2 and Figure 35.

**[Table 2]**

| **mM** | **1time** | **2time** | **3time** | **average** |
|---|---|---|---|---|
| **0.6** | **0.85** | **0.85** | **0.83** | **0.84** |
| **0.4** | **0.6** | **0.58** | **0.56** | **0.58** |
| **0.2** | **0.3** | **0.28** | **0.28** | **0.28** |
| **0.1** | **0.1** | **0.1** | **0.1** | **0.1** |

### [Acquisition of Michaelis-Menten plot of the substrate peptide]

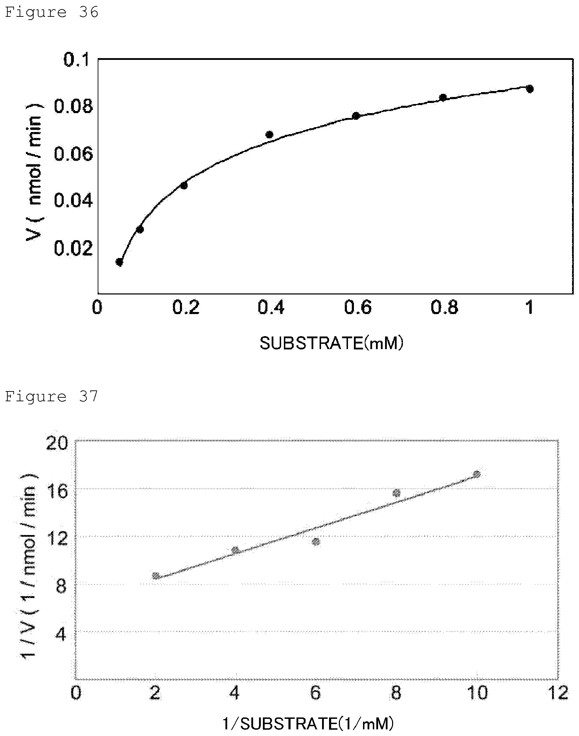

Into 1 mL of a 0.1 M phosphate buffer (pH 8.0, containing BSA (0.1 mg/mL)), 3.1 mg of the 14-residue peptide thus synthesized (SEQ ID NO:16) was dissolved to prepare a 2 mM solution. This solution was diluted to prepare substrate solutions having concentrations of 1.6 mM, 1.2 mM, 0.8 mM, 0.4 mM, 0.2 mM, 0.1 mM, and 0.05 mM. Separately, chymotrypsin (1 mg) was dissolved in 1 mL of a 0.1 M phosphate buffer (pH 8.0, containing BSA (0.1 mg/mL)). The resulting solution was diluted 10-fold, and further diluted 10-fold. The above operation was repeated so that a solution of 0.1 µg/mL was prepared. Into the same Eppendorf tube, 50 µ L of a substrate solution of each concentration that was sufficiently cooled on ice and 50 µL of the enzyme solution were transferred, followed by incubation for 30 minutes at 37°C. After 30 minutes, the reaction was terminated by addition of 10 µL of 1N hydrochloric acid. Then, 20 µL of the resulting reaction solution was mixed with 80 µL of buffer to make up a total of 100 µL, which was then measured by HPLC. A degradation rate per unit time (a reaction rate per unit time) was calculated from the peak area of HPLC of the reaction product (Figure 36). The reaction rate with respect to each substrate concentration is shown in Table 3.

**[Table 3]**

| **substrate(mM)** | **V (mol/min)** |
|---|---|
| **1** | **0.087** |
| **0.8** | **0.083** |
| **0.6** | **0.075** |
| **0.4** | **0.068** |
| **0.2** | **0.046** |
| **0.1** | **0.027** |
| **0.05** | **0.013** |

### [Acquisition of Lineweaver-Burk plot of the substrate peptide]

Into 1 mL of a 0.1 M phosphate buffer (pH 8.0, containing BSA (0.1 mg/mL)), 1.5 mg of the 14-residue peptide thus synthesized (SEQ ID NO:16) was dissolved to prepare a 1 mM solution. This solution was diluted to prepare substrate solutions having concentrations of 1 mM, 500 µM, 333 µM, 250 µM, and 200 µM. Separately, chymotrypsin (1 mg) was dissolved in 1 mL of a 0.1 M phosphate buffer (pH 8.0, containing BSA (0.1 mg/mL)). The resulting solution was diluted 10-fold, and further diluted 10-fold. The above operation was repeated so that a solution of 0.1 µg/mL was prepared. Into the same Eppendorf tube, 50 µ L of a substrate solution of each concentration that was sufficiently cooled on ice and 50 µL of the enzyme solution were transferred, followed by incubation for 30 minutes at 37°C. After 30 minutes, the reaction was terminated by addition of 10 µL of 1N hydrochloric acid. Then, 20 µL of the resulting reaction solution was mixed with 80 µL of buffer to make up a total of 100 µL, which was then measured by HPLC. A degradation rate per unit time (a reaction rate per unit time) was calculated from the peak area of HPLC of the reaction product (Figure 37). An inverse of the reaction rate with respect to an inverse of each substrate concentration is shown in Table 4.

**[Table 4]**

| **1/Substrate(1/mM)** | **1/V(1/mol/min)** |
|---|---|
| **2** | **7.02** |
| **4** | **8.51** |
| **6** | **11.9** |
| **8** | **10.9** |
| **10** | **14.7** |

### [Industrial Applicability]

The production method of the present invention enabled acquisition of a glycoprotein having a uniform amino acid sequence and sugar chain structure as well as a uniform higher order structure. Since the glycoprotein obtained by the production method of the present invention has a uniform higher order structure, not only are its blood half-life and intracellular transportation constant but also it uniformly has a p physiological activity. Further, according to the present invention, a mixture of glycoproteins can be controlled so as to have a desired physiological activity. Accordingly, the production method of the present invention is applicable particularly to the development of a pharmaceutical product utilizing a glycoprotein.

### [Sequence List Free Text]

SEQ ID NO: 1 is the amino acid sequence having a protecting group of Fragment 1.
SEQ ID NO:2 is the amino acid sequence having a benzyl thioester group of Fragment 1.
SEQ ID NO: 3 is the amino acid sequence having a protecting group of Fragment 2.
SEQ ID NO:4 is the glycosylated amino acid sequence having a protecting group of Fragment 2.
SEQ ID NO:5 is the glycosylated amino acid sequence having a benzyl thioester group and a protecting group of Fragment 2.
SEQ ID NO: 6 is the amino acid sequence having a protecting group of Fragment 3.
SEQ ID NO:7 is the amino acid of Fragment 3.
SEQ ID NO:8 is the glycosylated amino acid sequence having a protecting group.
SEQ ID NO:9 is a glycosylated amino acid sequence.
SEQ ID NO:10 is the glycosylated amino acid sequence of glycosylated OMSVP3.
SEQ ID NO:11 is the amino acid sequence having a protecting group of Fragment 2'.
SEQ ID NO: 12 is the amino acid sequence having a benzyl thioester group and a protecting group of Fragment 2'.
SEQ ID NO:13 is the amino acid sequence having a protecting group.
SEQ ID NO:14 is an amino acid sequence.
SEQ ID NO:15 is the amino acid sequence of non-glycosylated OMSVP3.
SEQ ID NO:16 is the amino acid sequence having a protecting group, which is a substrate of chymotrypsin.

## Claims

1. A method for producing a glycoprotein having uniform amino acid sequence, sugar chain structure, and higher order structure, comprising the following steps (a) to (c):
(a) folding a glycoprotein having uniform amino acid sequence and sugar chain to generate folded glycoproteins having plural kinds of higher order structures;
(b) fractionating the folded glycoproteins by column chromatography to obtain plural fractions containing said folded glycoproteins; and
(c) collecting a fraction having a predetermined activity from the fractions obtained by the step (b).

2. The method according to Claim 1, further comprising, after the step (c), the steps of:
(d) unfolding a glycoprotein contained in a fraction not collected in the step (c);
(e) refolding the unfolded glycoprotein;
(f) fractionating the refolded glycoprotein by column chromatography and collecting a fraction having a predetermined activity; and
(g) repeating the steps (d) to (f) as needed.

3. A method for screening for a glycoprotein having a predetermined physiological activity, comprising the following steps (i) to (iii):
(i) folding a glycoprotein having uniform amino acid sequence and sugar chain to generate folded glycoproteins having plural kinds of higher order structures;
(ii) fractionating the folded glycoproteins by column chromatography to obtain plural fractions containing said folded glycoproteins; and
(iii) measuring an activity of each of the fractions obtained by the step (ii) to determine whether or not it has a predetermined activity.

4. A method for obtaining a glycoprotein mixture having a desired physiological activity, comprising the following steps (A) to (D):
(A) folding a glycoprotein having uniform amino acid sequence and sugar chain to generate folded glycoproteins having plural kinds of higher order structures;
(B) fractionating the folded glycoproteins by column chromatography to obtain plural fractions containing said folded glycoproteins;
(C) measuring an activity of each of the fractions obtained by the step (B); and
(D) determining a mixing ratio of the fractions to obtain a desired activity and mixing the fractions according to the ratio thus obtained.

5. The method according to any one of Claims 1 to 4, wherein at least a part of the glycoprotein having uniform amino acid sequence and sugar chain are produced by a method comprising the following steps (1) to (6):
(1) esterifying a hydroxyl group of a resin having a hydroxyl group and a carboxyl group of an amino acid having an amino group protected with a fat-soluble protecting group or a carboxyl group of a glycosylated amino acid having an amino group protected with a fat-soluble protecting group;
(2) removing the fat-soluble protecting group to generate a free amino group;
(3) amidating the free amino group and a carboxyl group of an amino acid having an amino group protected with a fat-soluble protecting group or a carboxyl group of a glycosylated amino acid having an amino group protected with a fat-soluble protecting group;
(4) after the step (3), removing the fat-soluble protecting group to generate a free amino group;
(5) repeating the steps (3) and (4) once or more; and
(6) cleaving an ester bond formed in the step (1) by an acid.

6. The method according to Claim 5, wherein a part of the glycoprotein having uniform amino acid sequence and sugar chain are produced by the steps (1) to (6), and wherein the glycoprotein is produced by a method further comprising the following step (7):
(7) linking a part of the glycoprotein obtained in the step (6) with other peptides or glycopeptides by a ligation method.

## Patentansprüche

1. Verfahren zur Herstellung eines Glykoproteins mit einheitlicher Aminosäuresequenz, Zuckerkettenstruktur und Struktur höherer Ordnung, umfassend die folgenden Schritte (a) bis (c):
(a) Falten eines Glykoproteins mit einheitlicher Aminosäuresequenz und Zuckerkette, um gefaltete Glykoproteine mit mehreren Arten von Strukturen höherer Ordnung zu erzeugen;
(b) Fraktionieren der gefalteten Glykoproteine durch Säulenchromatographie, um mehrere Fraktionen zu erhalten, die die gefalteten Glykoproteine enthalten; und
(c) Sammeln einer Fraktion mit einer vorgegebenen Aktivität aus den durch den Schritt (b) erhaltenen Fraktionen.

2. Verfahren nach Anspruch 1, nach dem Schritt (c) weiterhin umfassend die Schritte:
(d) Auseindanderfalten eines Glykoproteins, das in einer Fraktion enthalten ist, die nicht in Schritt (c) gesammelt wurde;
(e) Neufalten des entfalteten Glykoproteins;
(f) Fraktionieren des neu gefalteten Glykoproteins durch Säulenchromatographie und Sammeln einer Fraktion mit einer vorgegebenen Aktivität; und
(g) Wiederholen der Schritte (d) bis (f) nach Bedarf.

3. Verfahren zum Screening auf ein Glykoprotein mit einer vorgegebenen physiologischen Aktivität, umfassend die folgenden Schritte (i) bis (iii):
(i) Falten eines Glykoproteins mit einheitlicher Aminosäuresequenz und Zuckerkette, um gefaltete Glykoproteine mit mehreren Arten von Strukturen höherer Ordnung zu erzeugen;
(ii) Fraktionieren der gefalteten Glykoproteine durch Säulenchromatographie, um mehrere Fraktionen zu erhalten, die die gefalteten Glykoproteine enthalten; und
(iii) Messung einer Aktivität einer jeden der durch Schritt (ii) erhaltenen Fraktionen, um zu bestimmen, ob sie eine vorgegebene Aktivität aufweist oder nicht.

4. Verfahren zur Gewinnung einer Glykoproteinmischung mit einer gewünschten physiologischen Aktivität, umfassend die folgenden Schritte (A) bis (D):
(A) Falten eines Glykoproteins mit einheitlicher Aminosäuresequenz und Zuckerkette, um gefaltete Glykoproteine mit mehreren Arten von Strukturen höherer Ordnung zu erzeugen;
(B) Fraktionieren der gefalteten Glykoproteine durch Säulenchromatographie, um mehrere Fraktionen zu erhalten, die die gefalteten Glykoproteine enthalten;
(C) Messen einer Aktivität jeder der durch den Schritt (B) erhaltenen Fraktionen; und
(D) Bestimmen eines Mischungsverhältnisses der Fraktionen, um eine gewünschte Aktivität zu erhalten, und Mischen der Fraktionen gemäß dem so erhaltenen Verhältnis.

5. Verfahren nach einem der Ansprüche 1 bis 4, wobei mindestens ein Teil des Glykoproteins mit einheitlicher Aminosäuresequenz und Zuckerkette durch ein Verfahren der folgenden Schritte (1) bis (6) hergestellt wird:
(1) Verestern einer Hydroxylgruppe eines Harzes mit Hydroxylgruppe und einer Carboxylgruppe einer Aminosäure mit einer Aminogruppe geschützt mit einer fettlöslichen Schutzgruppe, oder einer Carboxylgruppe einer glykosylierten Aminosäure mit einer Aminogruppe geschützt mit einer fettlöslichen Schutzgruppe;
(2) Entfernen der fettlöslichen Schutzgruppe, um eine freie Aminogruppe zu erzeugen;
(3) Amidieren der freien Aminogruppe und einer Carboxylgruppe einer Aminosäure mit einer Aminogruppe, die mit einer fettlöslichen Schutzgruppe geschützt ist, oder einer Carboxylgruppe einer glykosylierten Aminosäure mit einer Aminogruppe geschützt mit einer fettlöslichen Schutzgruppe;
(4) nach Schritt (3), Entfernen der fettlöslichen Schutzgruppe, um eine freie Aminogruppe zu erzeugen;
(5) Wiederholen der Schritte (3) und (4) einmal oder mehr; und
(6) Spalten einer in der Stufe (1) gebildeten Esterbindung durch eine Säure.

6. Verfahren nach Anspruch 5, wobei ein Teil des Glykoproteins mit einheitlicher Aminosäuresequenz und Zuckerkette durch die Schritte (1) bis (6) hergestellt wird, und wobei das Glykoprotein nach einem Verfahren hergestellt wird, das zudem den folgenden Schritt (7) umfasst:
(7) Verknüpfen eines Teils des in Schritt (6) erhaltenen Glykoproteins mit anderen Peptiden oder Glycopeptiden durch ein Ligationsverfahren.

## Revendications

1. Méthode de production d'une glycoprotéine ayant une structure uniforme en termes de séquence d'acides aminés et de chaîne sucrée et une structure d'un ordre supérieur, qui comprend les étapes (a) à (c) suivantes :
(a) repliement d'une glycoprotéine ayant une séquence d'acides aminés et une chaîne sucrée uniformes pour produire des glycoprotéines repliées ayant plusieurs types de structures d'ordre supérieur ;
(b) fractionnement des glycoprotéines repliées par chromatographie sur colonne pour obtenir plusieurs fractions contenant lesdites glycoprotéines repliées ; et
(c) recueil d'une fraction qui présente une activité prédéterminée à partir des fractions obtenues à l'étape (b).

2. Méthode selon la revendication 1 comprenant en outre, après l'étape (c), les étapes de :
(d) dépliement d'une glycoprotéine contenue dans une fraction non recueillie à l'étape (c) ;
(e) repliement de la glycoprotéine dépliée;
(f) fractionnement de la glycoprotéine repliée par chromatographie sur colonne et recueil d'une fraction ayant une activité prédéterminée ; et
(g) répétition des étapes (d) à (f) selon les besoins.

3. Méthode de triage d'une glycoprotéine ayant une activité physiologique prédéterminée, qui comprend les étapes (i) à (iii) suivantes :
(i) repliement d'une glycoprotéine ayant une séquence d'acides aminés et une chaîne sucrée uniformes pour produire des glycoprotéines repliées ayant plusieurs types de structures d'ordre supérieur ;
(ii) fractionnement des glycoprotéines repliées par chromatographie sur colonne pour obtenir plusieurs fractions contenant lesdites glycoprotéines repliées ; et
(iii) mesure d'une activité de chacune des fractions obtenues à l'étape (ii) pour déterminer si elles présentent ou non une activité prédéterminée.

4. Méthode permettant d'obtenir un mélange de glycoprotéines ayant une activité physiologique souhaitée, qui comprend les étapes (A) à (D) suivantes :
(A) repliement d'une glycoprotéine ayant une séquence d'acides aminés et une chaîne sucrée uniformes pour produire des glycoprotéines repliées ayant plusieurs types de structures d'ordre supérieur ;
(B) fractionnement des glycoprotéines repliées par chromatographie sur colonne pour obtenir plusieurs fractions contenant lesdites glycoprotéines repliées ;
(C) mesure d'une activité de chacune des fractions obtenues à l'étape (B) ; et
(D) détermination d'un rapport de mélange des fractions pour obtenir une activité souhaitée et mélange des fractions en fonction du rapport ainsi obtenu.

5. Méthode selon l'une quelconque des revendications 1 à 4, où au moins une partie de la glycoprotéine ayant une séquence d'acides aminés et une chaîne sucrée uniformes est produite par une méthode comprenant les étapes (1) à (6) suivantes :
(1) estérification d'un groupement hydroxyle d'une résine qui présente un groupement hydroxyle et un groupement carboxyle d'un acide aminé dans lequel un groupement amine est protégé par un groupement protecteur liposoluble ou un groupement carboxyle d'un acide aminé glycosylé dans lequel un groupement amine est protégé par un groupement protecteur liposoluble ;
(2) élimination du groupement protecteur liposoluble pour produire un groupement amine libre ;
(3) amidation du groupement amine libre et d'un groupement carboxyle d'un acide aminé dans lequel un groupement amine est protégé par un groupement protecteur liposoluble ou d'un groupement carboxyle d'un acide aminé glycosylé dans lequel un groupement amine est protégé par un groupement protecteur liposoluble ;
(4) après l'étape (3), élimination du groupement protecteur liposoluble pour produire un groupement amine libre ;
(5) répétition des étapes (3) et (4) une ou plusieurs fois ; et
(6) clivage par un acide d'une liaison ester formée à l'étape (1).

6. Méthode selon la revendication 5, où une partie de la glycoprotéine ayant une séquence d'acides aminés et une chaîne sucrée uniformes est produite par les étapes (1) à (6) et où la glycoprotéine est produite par une méthode qui comprend en outre l'étape (7) suivante :
(7) liaison d'une partie de la glycoprotéine obtenue à l'étape (6) avec d'autres peptides ou glycopeptides par une méthode de ligation.


### [Sequence Listing]

### SEQUENCE LISTING

<110> Otsuka Chemical Co., Ltd.
<120> Method of manufacturing glycoprotein and screening method of glycoprotein
<130> OCKP0803P1F
<150> JP 2008-211144
   <151> 2008-08-19
<150> JP 2009-029206
   <151> 2009-06-08
<160> 16
<170> PatentIn version 3.1
<210> 1
   <211> 23
   <212> PRT
   <213> silver pheasant
<220>
   <223> amino acid sequence having blocking groups (fragment 1)
<220>
   <221> MOD_RES
   <222> (1) .. (1)
   <223> Leu having blocking group Boc
<220>
   <221> MOD_RES
   <222> (5) .. (5)
   <223> Ser having blocking group tBu
<220>
   <221> MOD_RES
   <222> (7) .. (7)
   <223> Asp having blocking group OtBu
<220>
   <221> MOD_RES
   <222> (8) .. (8)
   <223> Cys having blocking group Trt
<220>
   <221> MOD_RES
   <222> (9) .. (9)
   <223> Ser having blocking group tBu
<220>
   <221> MOD_RES
   <222> (10) .. (10)
   <223> Glu having blocking group OtBu
<220>
   <221> MOD_RES
   <222> (11) .. (11)
   <223> Tyr having blocking group tBu
<220>
   <221> MOD_RES
   <222> (13) .. (13)
   <223> Lys having blocking group Boc
<220>
   <221> MOD_RES
   <222> (16) .. (16)
   <223> Cys having blocking group Trt
<220>
   <221> MOD_RES
   <222> (17)..(17)
   <223> Thr having blocking group tBu
<220>
   <221> MOD_RES
   <222> (19) .. (19)
   <223> Glu having blocking group OtBu
<220>
   <221> MOD_RES
   <222> (20) .. (20)
   <223> Tyr having blocking group tBu
<220>
   <221> MOD_RES
   <222> (21) .. (21)
   <223> Arg having blocking group Pbf
<400> 1
   Leu Ala Ala Val Ser Val Asp Cys Ser Glu Tyr Pro Lys Pro Ala
   Cys
   1               5                   10                  15
   Thr Met Glu Tyr Arg Pro Leu
               20
<210> 2
   <211> 23
   <212> PRT
   <213> silver pheasant
<220>
   <223> amino acid sequence having benzyl thioester group
   (fragment 1)
<220>
   <221> MOD_RES
   <222> (23) .. (23)
   <223> Leu having benzyl thioester group
<400> 2
   Leu Ala Ala Val Ser Val Asp Cys Ser Glu Tyr Pro Lys Pro Ala
   Cys
   1               5                   10                  15
   Thr Met Glu Tyr Arg Pro Leu
               20
<210> 3
   <211> 9
   <212> PRT
   <213> silver pheasant
<220>
   <223> amino acid sequence having blocking groups (fragment
   2)
<220>
   <221> MOD_RES
   <222> (1) .. (1)
   <223> Lys having blocking groups Fmoc and Boc
<220>
   <221> MOD_RES
   <222> (2)..(2)
   <223> Thr having blocking group tBu
<220>
   <221> MOD_RES
   <222> (3) .. (3)
   <223> Tyr having blocking group tBu
<220>
   <221> MOD_RES
   <222> (6) .. (6)
   <223> Lys having blocking group Boc
<220>
   <221> MOD_RES
   <222> (7) .. (7)
   <223> Cys having blocking group Trt
<400> 3
   Lys Thr Tyr Gly Asn Lys Cys Asn Phe
   1               5
<210> 4
   <211> 14
   <212> PRT
   <213> silver pheasant
<220>
   <223> glycosylated amino acid sequence having blocking
   groups
   (fragment 2)
<220>
   <221> MOD_RES
   <222> (1) .. (1)
   <223> Cys having blocking groups Boc and Thz
<220>
   <221> MOD_RES
   <222> (3) .. (3)
   <223> Ser having blocking group tBu
<220>
   <221> MOD_RES
   <222> (4) .. (4)
   <223> Asp having blocking group OtBu
<220>
   <221> MOD_RES
   <222> (5) .. (5)
   <223> Glycosylated Asn
<220>
   <221> MOD_RES
   <222> (6) .. (6)
   <223> Lys having blocking group Boc
<220>
   <221> MOD_RES
   <222> (7)..(7)
   <223> Thr having blocking group tBu
<220>
   <221> MOD_RES
   <222> (8) .. (8)
   <223> Tyr having blocking group tBu
<220>
   <221> MOD_RES
   <222> (11)..(11)
   <223> Lys having blocking group Boc
<220>
   <221> MOD_RES
   <222> (12)..(12)
   <223> Cys having blocking group Trt
<400> 4
   Cys Gly Ser Asp Asn Lys Thr Tyr Gly Asn Lys Cys Asn Phe
   1               5                   10
<210> 5
   <211> 14
   <212> PRT
   <213> silver pheasant
<220>
   <223> glycosylated amino acid sequence having benzyl thioester group
   and blocking group (fragment 2)
<220>
   <221> MOD_RES
   <222> (1) .. (1)
   <223> Cys having blocking group Thz
<220>
   <221> MOD_RES
   <222> (5) .. (5)
   <223> Glycosylated Asn
<220>
   <221> MOD_RES
   <222> (14) .. (14)
   <223> Phe having benzyl thioester group
<400> 5
   Cys Gly Ser Asp Asn Lys Thr Tyr Gly Asn Lys Cys Asn Phe
   1               5                   10
<210> 6
   <211> 19
   <212> PRT
   <213> silver pheasant
<220>
   <223> amino acid sequence having blocking groups (fragment
   3)
<220>
   <221> MOD_RES
   <222> (1) .. (1)
   <223> Cys having blocking group Trt
<220>
   <221> MOD_RES
   <222> (6) .. (6)
   <223> Glu having blocking group OtBu
<220>
   <221> MOD_RES
   <222> (7)..(7)
   <223> Ser having blocking group tBu
<220>
   <221> MOD_RES
   <222> (10) .. (10)
   <223> Thr having blocking group tBu
<220>
   <221> MOD_RES
   <222> (12)..(12)
   <223> Thr having blocking group tBu
<220>
   <221> MOD_RES
   <222> (14) .. (14)
   <223> Ser having blocking group tBu
<220>
   <221> MOD_RES
   <222> (15) .. (15)
   <223> His having blocking group Trt
<220>
   <221> MOD_RES
   <222> (18) .. (18)
   <223> Lys having blocking group Boc
<220>
   <221> MOD_RES
   <222> (19) .. (19)
   <223> Cys having blocking group Trt
<400> 6
   Cys Asn Ala Val Val Glu Ser Asn Gly Thr Leu Thr Leu Ser His
   Phe
   1               5                   10                  15
   Gly Lys Cys
<210> 7
   <211> 19
   <212> PRT
   <213> silver pheasant
<220>
   <223> amino acid sequence (fragment 3)
<400> 7
   Cys Asn Ala Val Val Glu Ser Asn Gly Thr Leu Thr Leu Ser His
   Phe
   1               5                   10                  15
   Gly Lys Cys
<210> 8
   <211> 33
   <212> PRT
   <213> silver pheasant
<220>
   <223> glycosylated amino acid sequence having blocking group
<220>
   <221> MOD_RES
   <222> (1) .. (1)
   <223> Cys having blocking group Thz
<220>
   <221> MOD_RES
   <222> (5) .. (5)
   <223> Glycosylated Asn
<400> 8
   Cys Gly Ser Asp Asn Lys Thr Tyr Gly Asn Lys Cys Asn Phe Cys Asn
   1 5 10 15
   Ala Val Val Glu Ser Asn Gly Thr Leu Thr Leu Ser His Phe Gly Lys
   20 25 30
   Cys
<210> 9
   <211> 33
   <212> PRT
   <213> silver pheasant
<220>
   <223> glycosylated amino acid sequence
<220>
   <221> MOD_RES
   <222> (5) .. (5)
   <223> Glycosylated Asn
<400> 9
   Cys Gly Ser Asp Asn Lys Thr Tyr Gly Asn Lys Cys Asn Phe Cys Asn
   1 5 10 15
   Ala Val Val Glu Ser Asn Gly Thr Leu Thr Leu Ser His Phe Gly Lys
   20 25 30
   Cys
<210> 10
   <211> 56
   <212> PRT
   <213> silver pheasant
<220>
   <223> glycosylated amino acid sequence (glycosylated OMSVP3)
<220>
   <221> MOD_RES
   <222> (28) .. (28)
   <223> Glycosylated Asn
<400> 10
<210> 11
   <211> 14
   <212> PRT
   <213> silver pheasant
<220>
   <223> amino acid sequence having blocking groups (fragment
   2 f)
<220>
   <221> MOD_RES
   <222> (1) .. (1)
   <223> Cys having blocking groups Boc and Thz
<220>
   <221> MOD_RES
   <222> (3) .. (3)
   <223> Ser having blocking group tBu
<220>
   <221> MOD_RES
   <222> (4) .. (4)
   <223> Asp having blocking group OtBu
<220>
   <221> MOD_RES
   <222> (6) .. (6)
   <223> Lys having blocking group Boc
<220>
   <221> MOD_RES
   <222> (7) .. (7)
   <223> Thr having blocking group tBu
<220>
   <221> MOD_RES
   <222> (8) .. (8)
   <223> Tyr having blocking group tBu
<220>
   <221> MOD_RES
   <222> (11)..(11)
   <223> Lys having blocking group Boc
<220>
   <221> MOD_RES
   <222> (12)..(12)
   <223> Cys having blocking group Trt
<400> 11
   Cys Gly Ser Asp Asn Lys Thr Tyr Gly Asn Lys Cys Asn Phe
   1               5                   10
<210> 12
   <211> 14
   <212> PRT
   <213> silver pheasant
<220>
   <223> amino acid sequence having benzyl thioester group and
   blocking
   group (fragment 2 f)
<220>
   <221> MOD_RES
   <222> (1) .. (1)
   <223> Cys having blocking group Thz
<220>
   <221> MOD_RES
   <222> (14) .. (14)
   <223> Phe having benzyl thioester group
<400> 12
   Cys Gly Ser Asp Asn Lys Thr Tyr Gly Asn Lys Cys Asn Phe
   1               5                   10
<210> 13
   <211> 33
   <212> PRT
   <213> silver pheasant
<220>
   <223> amino acid sequence having blocking group
<220>
   <221> MOD_RES
   <222> (1) .. (1)
   <223> Cys having blocking group Thz
<400> 13
   Cys Gly Ser Asp Asn Lys Thr Tyr Gly Asn Lys Cys Asn Phe Cys
   Asn
   1               5                   10                  15
   Ala Val Val Glu Ser Asn Gly Thr Leu Thr Leu Ser His Phe Gly
   Lys
               20                  25                  30
   Cys
<210> 14
   <211> 33
   <212> PRT
   <213> silver pheasant
<220>
   <223> amino acid sequence
<400> 14
   Cys Gly Ser Asp Asn Lys Thr Tyr Gly Asn Lys Cys Asn Phe Cys
   Asn
   1               5                   10                  15
   Ala Val Val Glu Ser Asn Gly Thr Leu Thr Leu Ser His Phe Gly
   Lys
               20                  25                  30
   Cys
<210> 15
   <211> 56
   <212> PRT
   <213> silver pheasant
<220>
   <223> amino acid sequence (non-glycosylated OMSVP3)
<400> 15
<210> 16
   <211> 14
   <212> PRT
   <213> artificial sequence
<220>
   <223> amino acid sequence having blocking group
   (substrate for chymotrypsin)
<220>
   <221> MOD_RES
   <222> (1) .. (1)
   <223> Cys having blocking group Thz
<400> 16
   Cys Gly Ser Asp Asn Lys Thr Tyr Gly Asn Lys Cys Asn Phe
   1               5                   10